# EUROPEAN PATENT APPLICATION

(11) **EP 3 173 788 A2**
(43) Date of publication of application: **31.05.2017**
(21) Application number: 17150231.3
(22) Date of filing: 14.03.2013
(51) Int. Cl.: G01N 33/535, G01N 33/569

(54) **MEANS AND METHODS FOR DIAGNOSTICS AND THERAPEUTICS OF DISEASES**

(30) Priority: 14.03.2012 US 201261610495 P
(62) Divisional of application: 13761756.9
(71) Applicant: Marx, Stephen, 97761 Jerusalem (IL)
(72) Inventor: Marx, Stephen, 97761 Jerusalem (IL)
(74) Representative: Lecomte & Partners

(57) **Abstract**

The present invention discloses a method of detecting T-cell proliferation in a subject comprising the steps of (a) obtaining a sample from said subject; and (b) profiling at least one parameter selected from the group consisting of: the activation level of GSK-3β, the expression level of GSK-3β and the activation or expression level of related members of pathways in which GSK-3β plays a part. It is a core aspect of the invention, wherein a significant deviation from normal values indicates cellular proliferation. The present invention further discloses kits for detecting T-cell proliferation.

## Description

### FIELD OF THE INVENTION

The present invention is in the field of diagnostic and therapeutic of pathological cell proliferation which is associated with variety of pathological conditions. More specifically, the invention is adapted to measure and regulate the expression and/or activation and inactivation levels of Glycogen Synthase Kinase 3β (GSK-3β) as a marker of T-cell proliferation.

### BACKGROUND OF THE INVENTION

Glycogen synthase kinase 3β (GSK-3β) is a serine/threonine kinase that was first isolated and purified as an enzyme capable of phosphorylating and inactivating the enzyme glycogen synthase. GSK-3β is now known to act as a downstream regulatory switch that determines the output of numerous signaling pathways initiated by diverse stimuli. Among these pathways GSK-3β is also suggested to play a role in cellular proliferation and apoptosis. The ability to detect the activation state of the GSK-3β and if necessary to regulate it, would allow a greater understanding and ability to treat and diagnose conditions related to cell proliferation and apoptosis.

In many conditions related to the immune response, including tissue rejection, inflammation, infection and Graft-versus-host disease (GVHD), there is a marked change in cell proliferation, in particular an increase in T-cell proliferation. Other conditions in which cell proliferation is a key factor includes cancer. A method of detecting, understanding and controlling these pathological states would greatly improve the diagnosis and treatment of these conditions.

The incidence of GVHD, for example, has increased substantially over recent years. The number of allogeneic hematopoietic stem cell transplants (HSCT) that occur annually continues to increase each year. Clinical observations along with current diagnostic techniques are time-consuming and require invasive tissue sampling, involving tissue biopsies obtained from an involved organ in order to confirm and diagnose diseases such as GVHD. Graft versus Host Disease (GVHD) is a life-threatening complication of HSCT occurring in 30 to 80% of patients (Sullivan K M 2004, Srinivasan R et al 2006).

Presently, the only way to diagnose GVHD is with biopsies of the affected tissue during phase 3 of the disorder, when T cells are already attacking the host tissues and there are observable clinical symptoms (Srinivasan R et al 2006, Jacobsohn DA and Vogelsang GB 2007).

Although biopsies are the only way to properly diagnose cell proliferation in diseases today, the diagnosis is determined only after T cells are already attacking the host tissues and the disease has reached phase 3 of the disorder, where clinical symptoms are apparent. This is time-consuming, invasive to the patient, and there is no guarantee that an accurate diagnosis can be rendered.

Biopsy procedures to procure tissue are thus invasive and incur delays of 24 to 48 hours before a diagnosis can be rendered. Furthermore, an unequivocal diagnosis is not always possible with biopsies, and diagnosis from clinical symptoms is not reliable, given that other post-transplant conditions may present in a similar manner. Therefore, currently, there is no available treatment that can effectively detect this disease before the onset of clinical symptoms.

There is therefore a long-felt need to provide a rapid, reliable, relatively non-invasive method, for detecting cell proliferation and other pathological syndromes in the early stages of the disease process.

### SUMMARY

The present invention provides a method for detecting T-cell proliferation in a subject comprising the steps of; (a) obtaining a sample from the subject; and (b) profiling at least one parameter selected from the group consisting of: the activation level of GSK-3β, the expression level of GSK-3β and the activation or expression level of related members of pathways in which GSK-3β plays a part; wherein a significant deviation from normal values indicates cellular proliferation.

It is another object of the current invention to disclose the method as defined in any of the above further comprises steps of profiling the expression level of p-GSK-3β or proteins associated with GSK-3β related pathways.

It is another object of the current invention to disclose the method as defined in any of the above, wherein a decrease in the expression level of serine 9 phosphorylated GSK-3β (p-GSK-3β) orGSK-3β or proteins associated with GSK-3β related pathways indicate increased T-cell proliferation.

It is another object of the current invention to disclose the method as defined in any of the above, further comprises steps of profiling the inactivation levels of GSK-3β or proteins associated with GSK-3β related pathways.

It is another object of the current invention to disclose the method as defined in any of the above further comprises steps of measuring protein kinase C (PKC) activity.

It is another object of the current invention to disclose the method as defined in any of the above further comprises steps of measuring protein kinase C (PKC) expression.

It is another object of the current invention to disclose the method as defined in any of the above, wherein a decrease in PKC expression indicates an increase in cell proliferation.

It is another object of the current invention to disclose the method as defined in any of the above further comprises steps of inhibiting PKC activity thereby significantly decreasing T-cell proliferation.

It is another object of the current invention to disclose the method as defined in any of the above further comprises steps of profiling GSK -3β related pathways selected from a group consisting of: total Gsk3β, ser9 phosphorylated Gsk3β, b-catenin, Dvl, tyr216 phosphorylated Gsk3β P38 MARK, Erk, PI-3K/Akt, PKC and any combination thereof.

It is another object of the current invention to disclose the method as defined in any of the above further comprises steps of inhibiting GSK -3β related pathways selected from a group consisting of: P38 MARK, Erk, PI-3K/Akt, PKC and any combination thereof.

It is another object of the current invention to disclose the method as defined in any of the above, wherein a decrease in cell proliferation is indicated when P38 MAPK, Erk and PI-3K/Akt, are inhibited and PKC is unaffected.

It is another object of the current invention to disclose the method as defined in any of the above, wherein inhibition of cell proliferation is indicated when P38 MAPK, Erk and PI-3K/Akt, are unaffected and PKC is inhibited.

It is another object of the current invention to disclose the method as defined in any of the above, wherein upregulation of cell proliferation is indicated when P38 MAPK, Erk and PI-3K/Akt, are inhibited and PKC is activated.

It is another object of the current invention to disclose the method as defined in any of the above, further comprises steps of measuring the phosphorylation levels of GSK-3β or proteins associated with GSK-3β related pathways.

It is another object of the current invention to disclose the method as defined in any of the above further comprises steps of identifying phosphorylation sites of GSK-3β or proteins associated with GSK-3β related pathways.

It is another object of the current invention to disclose the method as defined in any of the above further comprising steps of measuring phosphorylation of at least one of GSK -3β phosphorylation sites selected from a group consisting of: Serine 9, Thr324, Ser215, Ser219, Thr309, Thr356, Thr390, Thr392, Ser389 and any combination thereof.

It is another object of the current invention to disclose the method as defined in any of the above further comprising steps of measuring auto-phosphorylation of at least one of GSK -3β phosphorylation sites selected from a group consisting of: Serine 9, Thr324, Ser215, Ser219, Thr309, Thr356, Thr390, Thr392, Ser389 and any combination thereof.

It is another object of the current invention to disclose the method as defined in any of the above further comprising steps of sampling the spleen.

It is another object of the current invention to disclose the method as defined in any of the above further comprising steps of sampling the blood.

It is another object of the current invention to disclose the method as defined in any of the above further comprising steps of sampling a body fluid or tissue.

It is another object of the current invention to disclose the method as defined in any of the above further comprising steps of comparing the result of GSK-3β levels with a predetermined normal value obtained from healthy subjects.

It is another object of the current invention to disclose the method as defined in any of the above further comprising steps of comparing the result of GSK-3β status with a predetermined normal value obtained from healthy subjects, wherein a deviation of at least about 25 % from normal values indicates the presence of pathological T-cell proliferation.

It is another object of the current invention to disclose the method as defined in any of the above further comprising steps of detecting disorders associated with apoptosis, cell differentiation, protein synthesis and any combination thereof.

It is another object of the current invention to disclose the method as defined in any of the above further comprising steps of detecting disorders associated with apoptosis using annexin-V or anti-annexin-V mAb as a marker.

It is another object of the current invention to disclose the method as defined in any of the above further comprising the steps of profiling a predetermined disorder associated with pathological T-cell proliferation by the steps of: (a) preparing an array with predetermined antibodies or antigens recognizing GSK-3β or proteins associated with GSK-3β related pathways; (b) incubating the antibodies with a biological sample; (c) adding at least one labeled analyte or agent producing a signal; (d) detecting and analysing the signal produced by the analyte or agent using at least one fingerprinting or profiling technique; wherein a significant deviation from normal values indicates cellular proliferation.

It is another object of the current invention to disclose the method as defined in any of the above further comprising the steps of detecting and analysing the signal produced by the analyte or agent using at least one fingerprinting or profiling technique selected from a group consisting of:: Western blot, ELISA, immunohistochemistry, FACS, Mass spectrometry or any other conventionally used fingerprinting or profiling technique.

It is another object of the current invention to disclose the method as defined in any of the above further comprising steps of detecting pathological T-cell proliferation associated with Graft-versus-host disease (GVHD).

It is another object of the current invention to disclose the method as defined in any of the above further comprising steps of detecting GVHD at an early stage before appearance of pathological GVHD symptoms.

It is another object of the current invention to disclose the method as defined in any of the above further comprising steps of selecting GVHD symptoms from a group comprising Hunched posture, Closed eyes, Reduced activity, Diarrhea, Weight loss and any combination thereof.

It is another object of the current invention to disclose the method as defined in any of the above further comprising steps of detecting GVHD during phase 2 of a disease.

It is another object of the current invention to disclose the method as defined in any of the above further comprising steps of detecting GVHD at an early stage between days 4 and 5 after transplant.

It is another object of the current invention to disclose a method of detecting disorders associated with pathological T- cell proliferation, in a subject comprising the steps of; (a) obtaining a sample from a subject; (b) measuring the level of GSK-3β inactivation by measuring at least one parameter selected from a group consisting of: (i) serine 9 phosphorylation; (ii) the Wnt pathway activation; (iii) activated PKC and any combination thereof; comparing the result of the inactivation levels with a predetermined normal inactivation value obtained from healthy subjects; wherein a significant deviation from normal inactivation values indicates cellular proliferation.

It is another object of the current invention to disclose the method as defined in any of the above, wherein the step of measuring is carried out by measuring the serine 9 phosphorylation.

It is another object of the current invention to disclose the method as defined in any of the above, wherein the step of measuring the levels of GSK-3β inactivation, is carried out in a way selected form a group consisting of: (a) measuring β-catenin levels in the absence of serine 9 phosphorylation; (b) measuring the site of stimulation on the GSK-3β once stimulated through the activated Wnt pathway; (c) measuring a protein marker that is unique to the inactivation of GSK-3β through the activated Wnt pathway and any combination thereof.

It is another object of the current invention to disclose the method as defined in any of the above, wherein the step of measuring the levels of GSK-3β inactivation, is carried out in a way selected form a group consisting of: (a) measuring the site of stimulation on the GSK-3β once stimulated through activated PKC; (b) measuring a protein marker that is unique to the inactivation of GSK-3β through the activated PKC; and any combination thereof.

It is another object of the current invention to disclose a method of monitoring and regulating the GSK-3β activation state, comprising the steps of; (a) obtaining a biological sample from a subject; (b) determining at least one of: the expression level of GSK-3β, the activation level of GSK-3β, the expression or activation level of proteins associated with GSK-3β related pathways; (c) comparing the levels to control levels determined from healthy subjects; wherein levels with pre-specified characteristics is used for the diagnosis of pathological cell proliferation.

It is another object of the current invention to disclose a diagnostic kit for the detection of a disorder associated with pathological cell proliferation in a subject wherein the kit comprising profiling means adapted for profiling the status of GSK-3β or proteins associated with GSK-3β related pathways, in a biological sample derived from the subject.

It is another object of the current invention to disclose the diagnostic kit as defined in any of the above, wherein the status of GSK-3β or related proteins is selected from a group consisting of: (a) activation; (b) over-activation (c) expression levels; (d) inactivation; (e) over-inactivation and any combination thereof.

It is another object of the current invention to disclose the diagnostic kit as defined in any of the above, wherein the activation status of GSK-3β or related proteins (proteins associated with GSK-3β related pathways) is a determinant of the cell proliferation or cell apoptosis.

It is another object of the current invention to disclose the diagnostic kit as defined in any of the above, wherein the activation status of GSK-3β or related proteins (proteins associated with GSK-3β related pathways) is a determinant of apoptosis.

It is another object of the current invention to disclose the diagnostic kit as defined in any of the above, wherein the expression level of GSK-3β or related proteins (proteins associated with GSK-3β related pathways) is a determinant of the cell proliferation or cell apoptosis.

It is another object of the current invention to disclose the diagnostic kit as defined in any of the above, wherein the status is an indicator for the best treatment program.

It is another object of the current invention to disclose the diagnostic kit as defined in any of the above, wherein the profiling means are adapted to detect the activation status of GSK-3β or related proteins (proteins associated with GSK-3β related pathways).

It is another object of the current invention to disclose the diagnostic kit as defined in any of the above, wherein the profiling means are adapted to detect the expression level of GSK-3β or related proteins (proteins associated with GSK-3β related pathways).

It is another object of the current invention to disclose the diagnostic kit as defined in any of the above, wherein the activation status of GSK-3β or at least one related protein (proteins associated with GSK-3β related pathways) is a marker for cell proliferation.

It is another object of the current invention to disclose the diagnostic kit as defined in any of the above, wherein the activation status of GSK-3β is determined by profiling markers for the Wnt pathway or the actual site on the GSK-3β being affected by the activated Wnt pathway.

It is another object of the current invention to disclose the diagnostic kit as defined in any of the above, wherein the kit comprises profiling means for determining the activation status of GSK-3β is determined by profiling the status of at least one protein selected from a group including: total Gsk3β, PKC, b-catenin, Dvl, Erk, P38 MAPK, PI-3K/Akt, tyr216 phosphorylated GSK-3β, serine 9 phosphorylated GSK-3β and any combination thereof.

It is another object of the current invention to disclose the diagnostic kit as defined in any of the above, wherein the kit comprises profiling means for profiling the activation status of GSK-3β is determined by measuring PKC activation sites on GSK-3β.

It is another object of the current invention to disclose the diagnostic kit as defined in any of the above, wherein the kit comprises profiling means for profiling over-activation of GSK-3β determined by measuring tyrosine 216 phosphorylation.

It is another object of the current invention to disclose the diagnostic kit as defined in any of the above, wherein over-activation of GSK-3β is determined by measuring the expression levels of GSK-3β.

It is another object of the current invention to disclose the diagnostic kit as defined in any of the above, wherein the kit comprises profiling means for profiling or measuring phosphorylation of at least one of GSK -3β phosphorylation sites selected from a group comprising: Serine 9, Thr324 ,Ser215, Ser219, Thr309, Thr356, Thr390, Thr392, Ser389 and any combination thereof.

It is another object of the current invention to disclose the diagnostic kit as defined in any of the above, wherein the kit comprises profiling means for profiling or measuring auto-phosphorylation of at least one of GSK -3β phosphorylation sites including: Serine 9, Thr324, Ser215, Ser219, Thr309, Thr356, Thr390, Thr392, Ser389 and any combination thereof.

It is another object of the current invention to disclose the diagnostic kit as defined in any of the above, wherein the kit comprises profiling means for profiling or measuring GSK -3β related pathways including total Gsk3β, ser9 phosphorylated Gsk3β, b-catenin, Dvl, tyr216 phosphorylated Gsk3β P38 MARK, Erk, PI-3K/Akt, PKC and any combination thereof.

It is another object of the current invention to disclose the diagnostic kit as defined in any of the above, wherein the presence of over-inactivated GSK-3β as indicated by abnormal levels of phosphorylated serine 9 provides a positive diagnosis of pathological cell proliferation.

It is another object of the current invention to disclose the diagnostic kit as defined in any of the above, wherein the pathological cell proliferation is a marker for at least one pathological condition associated with cell proliferation, tissue rejection, cancer, wound healing, tissue regeneration, aging, skin care, obstetrics and any phenomena associated with abnormal levels of cellular proliferation or apoptosis.

It is another object of the current invention to disclose the diagnostic kit as defined in any of the above, wherein the kit is adapted for detecting disorders associated with apoptosis using annexin-V protein or anti-annexin-V mAb as a marker.

The diagnostic kit according to It is another object of the current invention to disclose the diagnostic kit as defined in any of the above 5, wherein the pathological cell proliferation is detectable during phase 2 of a GVHD disease, prior to appearance of disease symptoms.

It is another object of the current invention to disclose the diagnostic kit as defined in any of the above, wherein the GVHD symptoms include hunched posture, closed eyes, reduced activity, diarrhea and weight loss.

It is another object of the current invention to disclose the diagnostic kit as defined in any of the above, wherein the protein marker that is unique to the inactivation of GSK-3β is selected from a group comprising abnormal levels of phosphorylated serine 9 GSK-3β, abnormal levels of markers for Wnt activity including but not limited to abnormal levels of β-catenin expression, abnormal levels of stimulation at the site on the GSK-3β affected by the activated Wnt pathway, abnormal levels of protein markers unique to the inactivation of GSK-3β through the activated Wnt pathway, b-catenin, Dvl, tyr216 phosphorylated Gsk3β P38 MARK, Erk, PI-3K/Akt, PKC and any combination thereof.

It is another object of the current invention to disclose the diagnostic kit as defined in any of the above, comprising profiling means for detection of pathological or normal physiological conditions that are also characterized by increased cell proliferation, decreased cell apoptosis and an immune response.

It is another object of the current invention to disclose the diagnostic kit as defined in any of the above, wherein the results provided by the kit indicate the pathways to be modulated to control the activation status and/ or expression level of GSK-3β, thereby regulating T-cell proliferation.

It is another object of the current invention to disclose the diagnostic kit as defined in any of the above, wherein the kit is adapted for the detection and quantification of the activation or expression status of GSK-3β or related proteins (proteins associated with GSK-3β related pathways), further wherein the activation or expression status is an indicator for the progression/ presence of pathological T-cell proliferation.

It is another object of the current invention to disclose the diagnostic kit as defined in any of the above, wherein the pathological T-cell proliferation is diagnosed by determining the activation or expression status of GSK-3β or related proteins (proteins associated with GSK-3β related pathways) in samples from a subject selected from a group consisting of: (a) blood; (b) spleen; (c) biological fluid; (d) tissue and any combination thereof.

It is another object of the current invention to disclose a diagnostic kit for the detection of T-cell proliferation, provided with the means for detecting the presence of inactivated GSK-3β as indicated by abnormal levels of stimulation at the site on the GSK-3β affected by activated PKC, or as indicated by abnormal levels of a protein marker that is unique to the inactivation of GSK-3β.

It is another object of the current invention to disclose the diagnostic kit as defined in any of the above, wherein provided with the means for detecting the presence of over-inactivated GSK-3β as indicated by abnormal levels of phosphorylated serine 9 and abnormal levels of markers for Wnt activity including but not limited to abnormal levels of β-catenin expression, abnormal levels of stimulation at the site on the GSK-3β affected by the activated Wnt pathway, and abnormal levels of protein markers unique to the inactivation of GSK-3β through the activated Wnt pathway; and by abnormal levels of markers for activated PKC inactivation of the GSK-3β including but not limited to abnormal levels of stimulation at the site on the GSK-3β affected by the activated PKC, and abnormal levels of protein markers unique to the inactivation of GSK-3β through activated PKC; in combination, thereby providing a positive diagnosis of cell proliferation.

It is another object of the current invention to disclose a method of regulating T-cell proliferation in a patient, comprising the steps of monitoring the activation or expression status of GSK-3β or related proteins (proteins associated with GSK-3β related pathways) and modulating according to the needs of the patient.

It is another object of the current invention to disclose a method of diagnosing a disorder associated with pathological T-cell proliferation in a subject comprising the steps of: (a) measuring GSK-3β inactivation status or indicators thereof; (b) measuring GSK-3β activation status or indicators thereof; (c)combining measurements of activation and inactivation status or indicators thereof; wherein an activation to inactivation ratio is obtained characteristic of the disorder associated with pathological T-cell proliferation.

It is another object of the current invention to disclose the method as defined in any of the above, wherein the step (a) of determining inactivation is done by measuring at least one inactivation indicator selected from the group including serine 9 phosphorylation, Wnt activation as determined by β-catenin levels or through a protein marker specific for inactivation of the GSK-3β through the activated Wnt pathway or through the site on the GSK-3β that the activated Wnt pathway affects to stimulate inactivation of the GSK-3β; PKC inactivation sites on GSK-3β or through a protein marker specific for inactivation of the GSK-3β including Dvl, tyr216 phosphorylated Gsk3β P38 MARK, Erk, PI-3K/Akt, PKC, or any combination thereof.

It is another object of the current invention to disclose a protein chip comprising an array of bound antibodies, biomarkers or antigens useful for determining the expression levels of proteins associated with T-cell proliferation in a given sample.

It is another object of the current invention to disclose the protein chip as defined in any of the above, comprising antibodies, biomarkers or antigens characteristic of the activation state and/ or expression level of GSK-3β, related pathways, apoptosis markers, proliferation markers, and other biological and biochemical molecules associated with cell proliferation.

It is another object of the current invention to disclose the protein chip as defined in any of the above, wherein the antibodies, biomarkers or antigens characteristic of the activation state and/ or expression level of GSK-3β, related pathways, apoptosis markers, proliferation markers, and other biological and biochemical molecules associated with cell proliferation are detectable before the appearance of GVHD symptoms in the subject.

It is another object of the current invention to disclose the protein chip as defined in any of the above, wherein the antibodies, biomarkers or antigens are selected from the group comprising, but not limited to, total GSK-3β, ser 9 phosphorylated GSK-3β, ser 9 phosphorylated GSK-3β, PKC, phosphorylated PKC, b-catenin, Dvl, Akt, PI-3K/Akt Erk, P38 MAPK, Tyrosine 216 phosphorylated GSK-3β, Serum Albumin, markers for apoptosis comprising Fas, Bcl family, Cyctochrome C and caspases, markers for immune activity comprising Nf-kB and CD25, markers for proliferation comprising Cyclin D1, PCNA and p27), and any combination thereof.

It is another object of the current invention to disclose a method of regulating the activation status and/ or expression level of the GSK-3β in a in a subject comprising the steps of; (a) obtaining a blood sample from a patient or subject; (b) determining the activation status or expression level of GSK-3β or related proteins, (c) regulating the activation status or expression level of GSK-3β or related proteins according to the results of step (b); and (d) obtaining a normal activation status and/ or expression level of GSK-3β or related proteins; wherein regulation of the activation status and/ or expression level of the GSK-3β or related proteins is adapted for controlling a disorder associated with pathological T-cell proliferation such as GVHD.

It is another object of the current invention to disclose a diagnostic kit for the detection and quantification of at least one of: the activation status of GSK-3β, the expression status of GSK-3β and the activation or expression status of proteins associated with GSK-3β related pathways, wherein the status is an indicator of cell proliferation or apoptosis levels, further wherein the status is an indicator for the best treatment program.

It is another object of the current invention to disclose a regulatory and therapeutic kit, wherein the therapeutic molecule is GSK-3β or related proteins (proteins associated with GSK-3β related pathways).

It is another object of the current invention to disclose the kit as defined in any of the above, wherein the therapeutic kit is useful in applications selected from a group consisting of: GVHD, tissue rejection, cancer, wound healing, tissue regeneration, aging, skin care, obstetrics, autoimmune diseases and any phenomena associated with abnormal levels of cellular proliferation or apoptosis.

The following items are disclosed:
1. A method of detecting T-cell proliferation in a subject comprising the steps of;
   a. obtaining a sample from said subject; and
   b. profiling at least one parameter selected from the group consisting of:
      the activation level of GSK-3β, the expression level of GSK-3β and
      the activation or expression level of related members of pathways in which GSK-3β plays a part;
   wherein a significant deviation from normal values indicates cellular proliferation.
2. The method according to item 1 further comprises steps of profiling the expression level of p- GSK-3β or proteins associated with GSK-3β related pathways.
3. The method according to item 2 wherein a decrease in the expression level of serine 9 phosphorylated GSK-3β (p- GSK-3β) GSK-3b or proteins associated with GSK-3β related pathways indicate increased T-cell proliferation.
4. The method according to item 1, further comprises steps of profiling the inactivation levels of GSK-3β or proteins associated with GSK-3β related pathways.
5. The method according to item 1, further comprises steps of measuring protein kinase C (PKC) activity.
6. The method according to item 1, further comprises steps of measuring protein kinase C (PKC) expression.
7. The method according to item 6, wherein a decrease in PKC expression indicates an increase in cell proliferation.
8. The method according to item 6, further comprises steps of inhibiting PKC activity thereby significantly decreasing T-cell proliferation.
9. The method according to item 1, further comprises steps of profiling GSK - 3β related pathways selected from a group consisting of: total GSK-3β, ser9 phosphorylated GSK-3β, b-catenin, Dvl, tyr216 phosphorylated GSK-3b P38 MARK, Erk, PI-3K/Akt, PKC and any combination thereof.
10. The method according to item 1, further comprises steps of inhibiting GSK - 3β related pathways selected from a group consisting of: P38 MARK, Erk, PI-3K/Akt, PKC and any combination thereof.
11. The method according to item 9, wherein a decrease in cell proliferation is indicated when P38 MAPK, Erk and PI-3K/Akt, are inhibited and PKC is unaffected.
12. The method according to item 9, wherein inhibition of cell proliferation is indicated when P38 MAPK, Erk and PI-3K/Akt, are unaffected and PKC is inhibited.
13. The method according to item 9, wherein upregulation of cell proliferation is indicated when P38 MAPK, Erk and PI-3K/Akt, are inhibited and PKC is activated.
14. The method according to item 1, further comprises steps of measuring the phosphorylation levels of GSK-3b or proteins associated with GSK- 3β related pathways.
15. The method according to item 1 further comprises steps of identifying phosphorylation sites of GSK-3β or proteins associated with GSK- 3β related pathways.
16. The method according to item 14 further comprising steps of measuring phosphorylation of at least one of GSK -3β phosphorylation sites selected from a group consisting of: Serine 9, Thr324, Ser215, Ser219, Thr309, Thr356, Thr390, Thr392, Ser389 and any combination thereof.
17. The method according to item 14 further comprising steps of measuring auto-phosphorylation of at least one of GSK -3β phosphorylation sites selected from a group consisting of: Serine 9, Thr324, Ser215, Ser219, Thr309, Thr356, Thr390, Thr392, Ser389 and any combination thereof.
18. The method according to item 1 further comprising steps of sampling the spleen.
19. The method according to item 1 further comprising steps of sampling the blood.
20. The method according to item 1 further comprising steps of sampling a body fluid or tissue.
21. The method according to item 1 further comprising steps of comparing the result of said GSK-3β levels with a predetermined normal value obtained from healthy subjects.
22. The method according to item 1 further comprising steps of comparing the result of said GSK-3β status with a predetermined normal value obtained from healthy subjects, wherein a deviation of at least about 25 % from normal values indicates the presence of pathological T-cell proliferation.
23. The method according to item 1 further comprising steps of detecting disorders associated with apoptosis, cell differentiation, protein synthesis and any combination thereof.
24. The method according to item 24 further comprising steps of detecting disorders associated with apoptosis using annexin-V or anti-annexin-V mAb as a marker.
25. The method according to item 1 further comprising the steps of profiling a predetermined disorder associated with pathological T-cell proliferation by the steps of a. preparing an array with predetermined antibodies or antigens recognizing GSK-3β or proteins associated with GSK-3β related pathways; b. incubating said antibodies with a biological sample; c. adding at least one labeled analyte or agent producing a signal; d. detecting and analysing the signal produced by said analyte or agent using at least one fingerprinting or profiling technique, wherein a significant deviation from normal values indicates cellular proliferation.
26. The method according to item 25 further comprising the steps of detecting and analysing the signal produced by said analyte or agent using at least one fingerprinting or profiling technique selected from a group consisting of:: Western blot, ELISA, immunohistochemistry, FACS, Mass spectrometry or any other conventionally used fingerprinting or profiling technique.
27. The method according to item 1 further comprising steps of detecting pathological T-cell proliferation associated with Graft-versus-host disease (GVHD).
28. The method according to item 27 further comprising steps of detecting GVHD at an early stage before appearance of pathological GVHD symptoms.
29. The method according to item 28, further comprising steps of selecting GVHD symptoms from a group comprising Hunched posture, Closed eyes, Reduced activity, Diarrhea, Weight loss and any combination thereof.
30. The method according to item 27, further comprising steps of detecting GVHD during phase 2 of a disease.
31. The method according to item 27, further comprising steps of detecting GVHD at an early stage between days 4 and 5 after transplant.
32. A method of detecting disorders associated with pathological T- cell proliferation, in a subject comprising the steps of; a. obtaining a sample from a subject; b. measuring the level of GSK-3β inactivation by measuring at least one parameter selected from a group consisting of: (a) serine 9 phosphorylation; (b) the Wnt pathway activation; (c) activated PKC and any combination thereof; comparing the result of said inactivation levels with a predetermined normal inactivation value obtained from healthy subjects, wherein a significant deviation from normal inactivation values indicates cellular proliferation.
33. The method according to item 32, wherein said step of measuring is carried out by measuring said serine 9 phosphorylation..
34. The method according to item 32, wherein said step of measuring the levels of GSK-3β inactivation, is carried out in a way selected form a group consisting of: (a) measuring β-catenin levels in the absence of serine 9 phosphorylation; (b) measuring the site of stimulation on the GSK-3β once stimulated through the activated Wnt pathway; (c) measuring a protein marker that is unique to the inactivation of GSK-3β through the activated Wnt pathway and any combination thereof.
35. The method according to item 32, wherein said step of measuring the levels of GSK-3β inactivation, is carried out in a way selected form a group consisting of: (a) measuring the site of stimulation on the GSK-3β once stimulated through activated PKC; (b) measuring a protein marker that is unique to the inactivation of GSK-3β through the activated PKC; and any combination thereof.
36. A method of monitoring and regulating the GSK-3β activation state, comprising the steps of; a. obtaining a biological sample from a subject, b. determining at least one of: the expression level of GSK-3 , the activation level of GSK-3β, the expression or activation level of proteins associated with GSK-3β related pathways, c. comparing said levels to control levels determined from healthy subjects, wherein levels with pre-specified characteristics is used for the diagnosis of pathological cell proliferation.
37. A diagnostic kit for the detection of a disorder associated with pathological cell proliferation in a subject wherein said kit comprising profiling means adapted for profiling the status of GSK-3β or proteins associated with GSK-3β related pathways, in a biological sample derived from said subject.
38. The diagnostic kit according to item 37, wherein said status of GSK-3β or related proteins is selected from a group consisting of: (a) activation; (b) over-activation (c) expression levels; (d) inactivation; (e) over-inactivation and any combination thereof.
39. The diagnostic kit according to item 38, wherein the activation status of GSK-3β or related proteins (proteins associated with GSK-3β related pathways) is a determinant of said cell proliferation or cell apoptosis.
40. The diagnostic kit according to item 38, wherein the activation status of GSK-3β or related proteins (proteins associated with GSK-3β related pathways) is a determinant of apoptosis.
41. The diagnostic kit according to item 38, wherein the expression level of GSK-3β or related proteins (proteins associated with GSK-3β related pathways) is a determinant of said cell proliferation or cell apoptosis.
42. The diagnostic kit according to item 38, wherein said status is an indicator for the best treatment program.
43. The diagnostic kit according to item 38, wherein said profiling means are adapted to detect the activation status of GSK-3β or related proteins (proteins associated with GSK-3β related pathways).
44. The diagnostic kit according to item 38, wherein said profiling means are adapted to detect the expression level of GSK-3β or related proteins (proteins associated with GSK-3β related pathways).
45. The diagnostic kit according to item 38, wherein the activation status of GSK-3β or at least one related protein (proteins associated with GSK- 3β related pathways) is a marker for cell proliferation.
46. The diagnostic kit according to item 38, wherein the activation status of GSK-3β is determined by profiling markers for the Wnt pathway or the actual site on the GSK-3β being affected by the activated Wnt pathway.
47. The diagnostic kit according to item 38, wherein said kit comprises profiling means for determining the activation status of GSK-3β is determined by profiling the status of at least one protein selected from a group including: total GSK-3β, PKC, b-catenin, Dvl, Erk, P38 MAPK, PI- 3K/Akt, tyr216 phosphorylated GSK-3β, serine 9 phosphorylated GSK-3β and any combination thereof.
48. The diagnostic kit according to item 38, wherein said kit comprises profiling means for profiling the activation status of GSK-3β is determined by measuring PKC activation sites on GSK-3β.
49. The diagnostic kit according to item 38, wherein said kit comprises profiling means for profiling over-activation of GSK-3β determined by measuring tyrosine 216 phosphorylation.
50. The diagnostic kit according to item 38, wherein over-activation of GSK- 3β is determined by measuring the expression levels of GSK-3β.
51. The diagnostic kit according to item 38, wherein said kit comprises profiling means for profiling or measuring phosphorylation of at least one of GSK -3β phosphorylation sites selected from a group comprising: Serine 9, Thr324 ,Ser215, Ser219, Thr309, Thr356, Thr390, Thr392, Ser389 and any combination thereof.
52. The diagnostic kit according to item 38, wherein said kit comprises profiling means for profiling or measuring auto-phosphorylation of at least one of GSK -3β phosphorylation sites including: Serine 9, Thr324, Ser215, Ser219, Thr309, Thr356, Thr390, Thr392, Ser389 and any combination thereof.
53. The diagnostic kit according to item 38, wherein said kit comprises profiling means for profiling or measuring GSK -3β related pathways including total GSK-3β, ser9 phosphorylated Gsk3p, b-catenin, Dvl, tyr216 phosphorylated GSK-3β P38 MARK, Erk, PI-3K/Akt, PKC and any combination thereof.
54. The diagnostic kit according to item 38, wherein the presence of over-inactivated GSK-3β as indicated by abnormal levels of phosphorylated serine 9 provides a positive diagnosis of pathological cell proliferation.
55. The diagnostic kit according to item 38, wherein said pathological cell proliferation is a marker for at least one pathological condition associated with cell proliferation, tissue rejection, cancer, wound healing, tissue regeneration, aging, skin care, obstetrics and any phenomena associated with abnormal levels of cellular proliferation or apoptosis.
56. The diagnostic kit according to item 55, wherein said kit is adapted for detecting disorders associated with apoptosis using annexin-V protein or anti-annexin-V mAb as a marker.
57. The diagnostic kit according to item 55, wherein said pathological cell proliferation is detectable during phase 2 of a GVHD disease, prior to appearance of disease symptoms.
58. The diagnostic kit according to item 57, wherein said GVHD symptoms include hunched posture, closed eyes, reduced activity, diarrhea and weight loss.
59. The diagnostic kit according to item 55, wherein said protein marker that is unique to the inactivation of GSK-3β is selected from a group comprising abnormal levels of phosphorylated serine 9 GSK-3β, abnormal levels of markers for Wnt activity including but not limited to abnormal levels of β-catenin expression, abnormal levels of stimulation at the site on the GSK-3β affected by the activated Wnt pathway, abnormal levels of protein markers unique to the inactivation of GSK-3β through the activated Wnt pathway, b-catenin, Dvl, tyr216 phosphorylated GSK-3β P38 MARK, Erk, PI-3K/Akt, PKC and any combination thereof.
60. The diagnostic kit according to item 38, comprising profiling means for detection of pathological or normal physiological conditions that are also characterized by increased cell proliferation, decreased cell apoptosis and an immune response.
61. The diagnostic kit according to item 38, wherein the results provided by said kit indicate the pathways to be modulated to control the activation status and/ or expression level of GSK-3β, thereby regulating T-cell proliferation.
62. The diagnostic kit according to item 38, wherein said kit is adapted for the detection and quantification of the activation or expression status of GSK- 3β or related proteins (proteins associated with GSK-3β related pathways), further wherein said activation or expression status is an indicator for the progression/ presence of pathological T-cell proliferation.
63. The diagnostic kit according to item 39, wherein said pathological T-cell proliferation is diagnosed by determining the activation or expression status of GSK-3 or related proteins (proteins associated with GSK-3β related pathways) in samples from a subject selected from a group consisting of: (a) blood; (b) spleen; (c) biological fluid; (d) tissue and any combination thereof.
64. A diagnostic kit for the detection of T-cell proliferation, provided with the means for detecting the presence of inactivated GSK-3β as indicated by abnormal levels of stimulation at the site on the GSK-3β affected by activated PKC, or as indicated by abnormal levels of a protein marker that is unique to the inactivation of GSK-3β.
65. The diagnostic kit for the detection of T-cell proliferation according to item 64, provided with the means for detecting the presence of over-inactivated GSK-3β as indicated by abnormal levels ofphosphorylated serine 9 and abnormal levels of markers for Wnt activity including but not limited to abnormal levels of β-catenin expression, abnormal levels of stimulation at the site on the GSK-3β affected by the activated Wnt pathway, and abnormal levels of protein markers unique to the inactivation of GSK-3β through the activated Wnt pathway; and by abnormal levels of markers for activated PKC inactivation of the GSK-3β including but not limited to abnormal levels of stimulation at the site on the GSK-3β affected by the activated PKC, and abnormal levels of protein markers unique to the inactivation of GSK-3β through activated PKC; in combination, thereby providing a positive diagnosis of cell proliferation.
66. A method of regulating T-cell proliferation in a patient, comprising the steps of monitoring the activation or expression status of GSK-3β or related proteins (proteins associated with GSK-3β related pathways) and modulating according to the needs of the patient.
67. A method of diagnosing a disorder associated with pathological T-cell proliferation in a subject comprising the steps of; a. measuring GSK-3β inactivation status or indicators thereof, b. measuring GSK-3β activation status or indicators thereof, c. combining measurements of activation and inactivation status or indicators thereof, wherein an activation to inactivation ratio is obtained characteristic of said disorder associated with pathological T-cell proliferation.
68. The method according to item 68, wherein said step (a) of determining inactivation is done by measuring at least one inactivation indicator selected from the group including serine 9 phosphorylation, Wnt activation as determined by β-catenin levels or through a protein marker specific for inactivation of the GSK-3β through the activated Wnt pathway or through the site on the GSK-3β that the activated Wnt pathway affects to stimulate inactivation of the GSK-3β; PKC inactivation sites on **GSK-3** or through a protein marker specific for inactivation of the GSK-3P including Dvl, tyr216 phosphorylated GSK-3β P38 MARK, Erk, PI-3K/Akt, PKC, or any combination thereof.
69. A protein chip comprising an array of bound antibodies, biomarkers or antigens useful for determining the expression levels of proteins associated with T-cell proliferation in a given sample.
70. The protein chip according to item 69, comprising antibodies, biomarkers or antigens characteristic of the activation state and/ or expression level of GSK-3β, related pathways, apoptosis markers, proliferation markers, and other biological and biochemical molecules associated with cell proliferation.
71. The protein chip according to item 70, wherein said antibodies, biomarkers or antigens characteristic of the activation state and/ or expression level of GSK-3β, related pathways, apoptosis markers, proliferation markers, and other biological and biochemical molecules associated with cell proliferation are detectable before the appearance of GVHD symptoms in said subject.
72. The protein chip of item 70, wherein said antibodies, biomarkers or antigens are selected from the group comprising, but not limited to, total GSK-3β, ser 9 phosphorylated GSK-3β, ser 9 phosphorylated GSK-3β, PKC, phosphorylated PKC, b-catenin, Dvl, Akt, PI-3K/Akt Erk, P38 MAPK, Tyrosine 216 phosphorylated GSK-3β, Serum Albumin, markers for apoptosis comprising Fas, Bel family, Cyctochrome C and caspases, markers for immune activity comprising Nf-kB and CD25, markers for proliferation comprising Cyclin D1, PCNA and p27), and any combination thereof.
73. A method of regulating the activation status and/ or expression level of the GSK-3β in a in a subject comprising the steps of; a. obtaining a blood sample from a patient or subject, b. determining the activation status or expression level of GSK-3β or related proteins, c. regulating the activation status or expression level of GSK-3β or related proteins according to the results of step (b), and d. obtaining a normal activation status and/ or expression level of GSK- 3β or related proteins; wherein regulation of said activation status and/ or expression level of the GSK-3β or related proteins is adapted for controlling a disorder associated with pathological T-cell proliferation such as GVHD.
74. A diagnostic kit for the detection and quantification of at least one of: the activation status of GSK-3β, the expression status of GSK-3β and the activation or expression status of proteins associated with GSK-3β related pathways, wherein said status is an indicator of cell proliferation or apoptosis levels, further wherein said status is an indicator for the best treatment program.
75. A regulatory and therapeutic kit, wherein the therapeutic molecule is GSK- 3β or related proteins (proteins associated with GSK-3β related pathways).
76. The regulatory and therapeutic kit according to item 75, wherein said therapeutic kit is useful in applications selected from a group consisting of: GVHD, tissue rejection, cancer, wound healing, tissue regeneration, aging, skin care, obstetrics, autoimmunediseases and any phenomena associated with abnormal levels of cellular proliferation or apoptosis.

### BRIEF DESCRIPTION OF THE DRAWINGS

The invention is herein described, by way of example only, with reference to the accompanying drawings. The particulars shown are by way of example only and for the purposes of illustrative discussion of the preferred embodiments of the present invention only. The description taken with the drawings should make apparent to those skilled in the art how several forms of the invention may be embodied in practice.

In the drawings:
**Figure 1** is a graphical representation of appearance of GVHD symptoms by time in GVHD model mice;
**Figure 2A** is a graphical representation of ser 9- phosphorylated GSK-3β (p- GSK-3β) protein expression levels in spleen samples;
**Figure 2B** is a graphical representation of total GSK-3β protein expression levels in spleen samples;
**Figure 3** is a graphical representation of phosphorylated PKC (p-PKC) protein expression levels in spleen samples;
**Figure 4** is a graphical representation showing the effect of LiCl on T-cell proliferation of splenocytes derived of treated and untreated Balb/c and C57BL/6 mice.
**Figure 5** is a graphical representation showing percentage of apoptosis in the GVHD model mice;
**Figure 6A** is a graphical representation of ser 9- phosphorylated GSK-3β (p- GSK-3β) protein expression levels in blood samples;
**Figure 6B** is a graphical representation of total GSK-3β protein expression levels in blood samples;
**Figure 7** is a flow diagram illustrating the preparation and analysis steps connecting a diagnostic kit according to specific embodiments of the present invention;
**Figure 8** is a graphical representation showing PKC's ability to regulate proliferation through the GSK-3β pathway in T-cells.

### DETAILED DESCRIPTION OF THE INVENTION

The following description is provided, alongside all chapters of the present invention, so as to enable any person skilled in the art to make use of said invention and sets forth the best modes contemplated by the inventor of carrying out this invention. Various modifications, however, is adapted to remain apparent to those skilled in the art, since the generic principles of the present invention have been defined specifically to provide means and methods for detecting T-cell proliferation in a subject.

The present invention solves many of the problems of current methods and systems for monitoring patients suffering from conditions associated with pathological cell proliferation and pathological cell apoptosis such as Graft-versus-host disease (GVHD).

The term **"expression level"** refers hereinafter to the amount of a protein or the amount of a protein that underwent a post-translational modification (e.g. phosphorylation).

The terms **"GSK-3β related proteins"** or **"GSK-3β related members"** refer hereinafter to proteins associated with GSK-3β related pathways, including antibodies, antigens or biomarkers associated with GSK-3β, proteins upstream of GSK-3β and GSK-3β substrates and downstream products i.e. described *inter alia* hereinafter.

It is therefore a preferred embodiment of the present invention to provide means and methods capable of detecting the expression level of glycogen synthase kinase 3β (GSK-3β) and related pathways. In this embodiment the expression and or activation status of GSK-3β and/or proteins associated with related pathways are surprisingly found to an indicator of pathological cell proliferation and medical disorders associated with pathological cell proliferation such as GVHD in a patient.

Thus in one embodiment, a main concept of the present invention is monitoring the different aspects of GSK-3β.

A further embodiment of the invention is the identification of phosphorylation sites of GSK-3β and their affect on the activation state of the aforementioned molecule.

A further embodiment of the invention is the monitoring of T-cell proliferation and T-cell apoptosis to monitor T-cell proliferation the and thus detecting early onset of GVHD and preventing the need for the invasive biopsy and surgery procedures. The methods and kits provided by the present invention enable a prompt early diagnosis, followed by timely initiation of appropriate immunosupressive therapy, which is vital for the reduction of morbidity and mortality associated with GVHD.

A further embodiment of the invention is the use of fingerprinting technology to diagnose multiple disorders associated with abnormal levels of cell proliferation. Such a fingerprinting technology is preferably based upon custom made protein chip and unique software.

According to a main embodiment of the invention, a diagnostic kit is provided that is adapted for the detection of a disorder associated with pathological cell proliferation, wherein the diagnostic molecule is GSK-3β and/or at least one protein associated with GSK-3β related pathways.

According to a further embodiment of the invention the diagnostic kit as described above comprises profiling means adapted for profiling the status of GSK-3β and/or related proteins (proteins associated with GSK-3β related pathways) in a biological sample derived from a subject or patient.

According to a further embodiment of the invention a diagnostic kit as described above is useful for the early detection of GVHD. GVHD serves herein as an example for a cell proliferative disorder that is firstly shown by the present invention to be associated with GSK-3β status and/or related pathways thereof.

It is herein acknowledged that GVHD has 3 molecular phases:
Molecular phase 1: activation of antigen presenting cells (APCs).
Molecular phase 2: T-cell activation, an increase in T-cell proliferation, T-cell differentiation and T cell migration.
Molecular phase 3: T cells target the tissue for destruction

Thus phase two of the disease is characterized by several activities on the cellular/molecular level including increased T-cell proliferation.

It is emphasized that GVHD development is not evident until clinical symptoms appear (phase three), when the patient's life is already in danger.

Thus advantages of a diagnostic kit as provided by the present invention may include:
- Enabling doctors to identify the onset of GVHD before symptoms appear.
- Administering a non-invasive diagnosis process which includes a simple series of blood tests and not an invasive biopsy.
- Early diagnosis of the disease, which significantly improves survival rates.
- Allowing disease management at an earlier stage, and possibly preventing the disease.
- Substantial cost reductions to the patient and medical institutions.

A main aspect of the invention is the ability to identify, monitor and then regulate the change in the GSK-3β molecule. This process comprises the flowing steps:
(a) Detecting a change in GSK-3β activation and/or expression state and/ or proteins associated with related pathways thereof, relative to control values in a biological sample derived of a subject or a patient;
(b) Monitoring and regulating the different pathways that affect the activation and/or expression state of GSK-3β;
(c) Regulating cellular proliferation and apoptosis in the subject or patient.

The following examples are specific to particular diseases, however, it will become apparent to anyone skilled in the art, after studying the present invention and examples described herein, other diseases and syndromes to which, with no undue burden can be predictably and reasonably diagnosed or treated. The disclosure shows by example, that activation or expression levels of GSK-3β or related members of pathways in which GSK-3β plays a part are related to T-cell proliferation. Since T cell proliferation is a significant phase, stage or process in many diseases, preparation of specific diagnostic kits, research tools treatment protocols, drug screening protocols, new drugs will now become predictable using the methodologies of the present invention. These methodologies are taught by the disclosure and examples described herein, and broadly speaking include:
a. obtaining a sample from a subject;
b. profiling the activation or expression level of GSK-3β or any related members of pathways in which GSK-3β plays a role; and,
c. measuring a significant deviation from normal values.

The deviation indicates an abnormality related to the disease in question.

According to one embodiment of the invention, it is demonstrated for the first time that GSK-3β regulates GVHD through the regulation of T-cell proliferation.

According to a further embodiment, the present invention shows that GSK-3β regulates T-cell proliferation through changes in the activation and/or expression state of GSK-3β and through specific substrates and their particular products. The function of GSK-3β is determined by its substrates and their particular products; the activation and/or expression state of GSK-3β initiates its function.

According to a further embodiment, the present invention identifies the expression and/or activation state (activation/inactivation, and specific changes in the activation state) and substrates with their particular products that regulate T-cell proliferation in association with the onset and progression of medical disorders associated with abnormal cell proliferation such as the GVHD disorder. It is herein shown for the first time that GSK-3β is connected to the onset and progression of GVHD.

According to a further embodiment of the present invention, a protein chip based on GSK-3β expression and/or activation state and associated substrates/products is provided.

Such a protein chip is adapted to monitor changes in the expression and/or activation state of GSK-3β and to monitor changes in the substrates of GSK-3β and their particular products. This allows for example, monitoring the progression of the state of the transplant and detecting the early onset of GVHD through monitoring changes in T-cell proliferation.

In a further embodiment, a protein chip based on GSK-3β activation state and associated substrates/products is provided. Such a kit is capable of monitoring changes in the activation state of GSK-3β and monitoring changes in the substrates of GSK-3β and their particular products. This allows monitoring the progression of the state of the transplant and detects the early onset of GVHD through monitoring changes in T-cell proliferation associated with changes in the GSK-3β activation state and GSK-3β substrates and their particular products which are associated with the onset and progression of GVHD. A protein chip (i.e. antibody microarray /protein microarray/ biomarker microarray/ antigen microarray) is used as a diagnostic tool

According to a further embodiment, the present invention shows that inactivation of GSK-3β stimulates an increase in T-cell proliferation and a decrease in T-cell apoptosis, while activation of GSK-3β stimulates a decrease in T-cell proliferation and an increase in T-cell apoptosis.

The technology provided by the present invention allows monitoring increases in T-cell proliferation which can be utilized as an indicator for the early onset of GVHD. The detection of the onset of GVHD is made during molecular phase 2 of the disorder which is associated with an increase in T cell proliferation.

The present invention gives detection and diagnosis manners of cell proliferation which can give a solution of problems of current methods and systems for monitoring patients suffering from conditions associated with pathological cell proliferation and pathological cell apoptosis.

It is therefore a preferred embodiment of the present invention to disclose a diagnostic kit, capable of detecting the expression and/or activation status of glycogen synthase kinase 3β (GSK-3β). In this embodiment the GSK-3β level of expression, activation and inactivation is an indicator of cellular activity in the patient.

GSK-3β plays a central role in regulating the cellular cycle, cellular proliferation and apoptosis through changes in its activation state. Determining the activation state of GSK-3β is therefore a useful tool for detecting and regulating both normal (physiological) and abnormal (pathological) cellular proliferation and apoptosis. GSK-3β is now known to act as a downstream regulatory switch that determines the output of numerous signaling pathways initiated by diverse stimuli (Frame and Cohen 2001, Grimes and Jope 2001, Woodgett 2001 and Doble and Woodgett 2003).

Thus in this embodiment the products produced through the activation or inactivation of the GSK-3β include both proliferatory and apoptotic factors. The activation status of the GSK-3β, the expression levels of the GSK-3β or the site on the GSK-3β responsible for activating or inactivating of the GSK-3β, affect cellular proliferation and / or apoptosis based on the products produced depending upon the conformation change of the GSK-3β.

Gsk3β has sites which are phosphorylated by PKC, therefore, they may play a role in Gsk3β regulation. These sites can be potentially used to monitor the activation state of Gsk3β, which can be used for diagnostic and therapeutic purposes. PKC works through Gsk3β to regulate T-cell proliferation. Therefore PKC can potentially be used to help more accurately assay Gsk3β activity levels, T-cell proliferation, PKC inhibition and inhibition of other pathways which regulate GSK3beta. This suggests that PKC inhibition and inhibition of other pathways which regulate GSK3beta activity play an important role in regulating proliferation. Further more, PKC inhibition and inhibition of other pathways which regulate GSK3beta activity play an important role in regulating cellular proliferation in the human body which suggests importance in developing diagnostics to identify and monitor normal and abnormal cellular proliferation in the human body and developing pharmaceutical to regulate normal and treat abnormal pathological levels of cellular proliferation in the body. PKC works through the GSK3beta to regulate T-cell proliferation. PKC regulates proliferation either working independently but synergistically with the GSK-3beta, or directly by regulating the activity / activation state of the GSK3beta. Through monitoring the activation state of the GSK3beta and the expression levels of the GSK3beta, one can identify, monitor and quantitate changes in cellular proliferation and apoptosis. Once one identify how the activation state the GSK3beta has been altered one can then regulate changes in cellular proliferation and apoptosis through regulating changes in the activation state of the GSK3beta.

In a further embodiment of the present invention, GSK-3β inactivation may also occur through the Wnt signaling pathway. Wnt activation inactivates the GSK-3β through an inactivation site independent of serine 9 phosphorylation and is identified and quantitated through measuring changes in non-phosphorylated β-catenin levels. In a further embodiment activation of the GSK-3β is either through dephosphorylation or through phosphorylation of tyrosine 216 (Y216). GSK-3β phosphorylation can be identified and quantitated through changes in the molecular weight of the GSK-3β using methods described forthwith in the description. In one aspect of this embodiment the diagnostic kit of the present invention uses antibodies to identify and quantify GSK-3β phosphorylation levels.

The activation status of GSK-3β is an indicator of cell proliferation and cell apoptosis in the patient. It is therefore a preferred embodiment of the present invention that GSK-3β will be used as diagnostic and/or therapeutic molecule incorporated into a diagnostic and/ or therapeutic kit for detection and monitoring of cell proliferation.

It is a further embodiment of the present invention that inactivation of GSK-3β through the serine 9 position increases T-cell proliferation. In another aspect of the present invention, inhibition of serine 9 phosphorylation in GSK-3β decreases T-cell proliferation. Thus in this embodiment T-cell proliferation and apoptosis can be regulated through activation or inactivation of GSK-3β by phosphorylation or dephosphorylation of the serine 9 position of GSK-3β.

It is a preferred embodiment of the diagnostic kit of the present invention to characterize GSK3β activity by detecting and quantifying GSK3β activation and inactivation at the aforementioned sites. Also in this embodiment, the diagnostic kit is able to detect and quantify GSK3β inactivation through activated protein kinase C (PKC) and GSK3β inactivation through activated Wnt.

Thus it is a preferred embodiment of the present invention to identify the site on the GSK-3β that is affected by the activated Wnt pathway and the site on the GSK-3β that is stimulated by activated PKC.

Another preferred embodiment of the present invention is to identify GSK-3β as a central molecule for the identification and regulation of normal and abnormal levels of cellular proliferation and apoptosis. In this embodiment regulation of normal and abnormal level of cellular proliferation and apoptosis is achieved through regulating the activation state of the GSK-3β.

Abnormal cell proliferation and apoptosis is a feature of pathological conditions such as Graft Versus Host Disease (GVHD) and other conditions related to immune responses in which there is proliferation of T-cells as part of the patient's response to a foreign body or bodies. In this embodiment and other preferred embodiments the diagnostic molecule and kit has also applications in GVHD, tissue rejection, cancer and any other phenomena or condition in which there is a change in cellular proliferation and/or apoptosis.

It is a further embodiment of the present invention that regulation of GSK-3β activation status by identification and quantification of inactivation and activation sites on GSK-3β and their associated pathways is adapted to regulate normal physiological and abnormal pathological levels of cellular proliferation and apoptosis. In this embodiment GSK-3β contains a plurality of activation and inactivation sites that can be identified and quantified by the diagnostic kit of the present invention.

A diagnostic kit of identifying cell proliferation is been preformed in at least phase 2 of a disease by identifying and quantifying the GSK-3β activation status, is a further embodiment of the present invention. In this embodiment, the diagnostic kit measures a range of parameters associated with GSK-3β and/or related proteins, activation and expression states and associated pathways. In a further embodiment, the aforementioned measurements are compared to the deviation from standard measurements taken from healthy subjects in order to determine the extent of cell proliferation and apoptosis. Furthermore in this embodiment the diagnostic kit and methods of the present invention will be able to detect T cell proliferation and apoptosis by determining how the GSK-3β regulates the aforementioned proliferation and apoptosis. In this aspect of the preferred embodiment the information provided by the diagnostic kit is used for determining the most effective treatment for a pathological conditions via the T cells through regulating activation and/or inactivation sites on the GSK-3β.

It is a further preferred embodiment of the present invention that the diagnostic kit is adapted to detect protein profiles specific and uniquely characteristic to pathological conditions and other unique protein profiles characteristic of related disorders so as to be able to differentiate between said related disorders and to make a correct and accurate diagnosis of GVHD. In this embodiment the unique protein profile prevents the diagnosis of false positive abnormal T-cell proliferation pathologies such as GVHD.

It is therefore a preferred embodiment of the present invention that GSK-3β acts as a regulatory and therapeutic molecule in a therapeutic or pharmaceutical kit in applications not limited to GVHD, tissue rejection, cancer, wound healing, tissue regeneration, aging, skin care, obstetrics and any phenomena associated with abnormal levels of cellular proliferation and/or apoptosis.

It is an embodiment of the present invention that a diagnostic kit capable of detecting and quantitating the activation status of the GSK-3β (GSK-3β activation levels and GSK-3β inactivation levels) is able to diagnose cell proliferation which occurs during phase II of a disease. The novel diagnostic kit is able to diagnose cell proliferation at a relatively early stage before extensive tissue damage occurs, in a quick and relatively non-invasive manner.

In a further embodiment of the present invention, the diagnostic kit for cell proliferation is able to determine the activation status of GSK-3β as measured by examining GSK-3β expression, the activation sites of the GSK-3β and the inactivation sites of the GSK-3β.

In another preferred embodiment of the present invention, the diagnostic and therapeutic kit is incorporated in an implant type device inserted into the patient's body at a site where constant monitoring and treatment of GSK-3β fluctuations is possible. In this embodiment the diagnostic implant is provided with the means to communicate GSK-3β fluctuations wirelessly and at frequent intervals, for example with radio-frequency identification (RFID) tags and readers or from a remote location using wireless internet technology. Thus for example a patient, having undergone a transplant operation may be released from a hospital to their own home with said diagnostic implant inserted and constantly transmitting data to doctors and medical staff at a remote location, any changes would be flagged up early and the patient then recalled for further monitoring. Also in this embodiment the diagnostic implant may further incorporate a therapeutic portion, in which the implant is able to calculate and dose the correct amount of GSK-3β upon detection of abnormal GSK-3β activity or to wirelessly receive dosing instructions from a medical professional monitoring the diagnostic implant.

In another embodiment of the invention, a protein chip utilizing an array of antibodies bound to a glass slide is used to determine the expression levels of different GVHD relevant proteins in a given sample.

In another embodiment of the invention, a diagnostic kit based on a protein chip is used to diagnose GVHD before the appearance of clinical symptoms. In certain embodiments the kit contains a custom-made protein chip comprising antibodies which are adapted to monitor the activation state and/ or expression level of the GSK-3β, related pathways, apoptosis markers, proliferation markers, and other biological and biochemical molecules. Such molecule candidates can be pre determined and analysed by western blot, FACS, and other fingerprinting techniques.

In another embodiment another plat form is the substrate of the GSK which determines the function of the GSK-3beta. GSK-3beta has at least 50 substrates which have been identified. The functions of the substrates are based on the products they produce. The function of the different substrates can be mapped out and connect to the products they produce .Another aspect that may need to be considered in the future is that the affect of the substrate may be determined by the cell / tissue type were it is located. The cell / tissue type may effect what products are being produced by the substrate in the cell / tissue in which it is located. By knowing the substrates that are present in your system, cellular function the GSK3beta is playing in the system is been diagnosed.

The diagnostic kit then can be matched to the activation state of the GSK3b, to the substrate, to the products, to the disorder, that is being produced. There may be multiple substrates that can trigger the same disorder or a closely related disorder. It may allow differentiation between similar disorders that was either very difficult or impossible prior to the plat form technology. It is a way to categorize disorders based on their substrates and products.

In another embodiment another plat form is finger printing. Every disorder has a specific finger print. Something unique to that particular disorder therefore each diagnostic kit has a specific finger printer for a specific disorder.

The protein level is examined with Mass Spectrometry so that all the proteins are presented in the system. Changes in protein expression and changes in phosphorylation of specific proteins are assessed. The RNA level is examined with RNA arrays and / or microRNA arrays to assess gene expression. The DNA level is examined, for example with SNP arrays to assess mutations.

The finger print could be on one level of complexity, multiple levels of complexity or involve all levels of complexity. It could be based on increased expression, decreased expression, or whether or not it is or is not expressed. It could be a combination of all these factors at a single time point or changes observed at multiple points over time. Another fingerprinting technique that can be used to study T-cell proliferation, Gsk3β and PKC in living cells is western blotting. Western blotting confirms the effectiveness of the inhibitors and activators by detecting the following proteins in a given sample. Using a fingerprinting technique (i.e. Western blot), the levels of proteins, antigens or biomarkers, exemplified in the list below can be determined:
- Total Gsk3β protein
- Gsk3β ser9 phosphorylation - indicates Gsk3β inactivation
- PKC Activation - indication of Gsk3β inactivation
- b-catenin - a measure of the inactivation of the Gsk3β through the activated Wnt Pathway
- Dvl - a protein upstream of Gsk3β in the Wnt pathway, and an indicator of Wnt pathway activation
- Erk - Map kinase signaling cascade protein that directly phosphorylates Gsk3β at Ser9.
- P38 MAPK - Map kinase signaling cascade protein that directly phosphorylates Gsk3β at Ser389.
- Gsk3β tyr216 Phosphorylation - Indicates activation of Gsk3β
- House keeping protein - loading control

It is included within the scope of the present invention that examples of disorders associated with pathological cell proliferation are herein listed:

### Diseases Associated with Proliferation and Apoptosis:

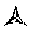 Autoimmune disease/disorder
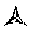 Cancer
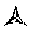 Hematological diseases/disorders
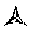 Pulmonary & Respiratory diseases/disorders
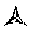 Cardiovascular diseases/disorders
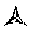 Gastrointestinal diseases/disorders
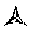 Ear, Nose, & Throat diseases/disorders
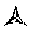 Cutaneous/dermatologic diseases/disorders
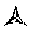 Immunological/Immunodefieiency disorders/disease
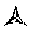 Allergic diseases/disorders
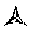 Endocrine diseases/disorders
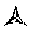 Genitourinary diseases/disorders
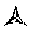 Metabolic diseases/disorders
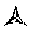 Kidney & Renal disease/disorders
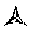 Hepatic & Biliary disease/disorders
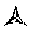 Mitochondrial diseases/disorders
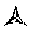 Eye and Vision diseases/disorders
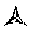 Temperomandibular disease/disorders
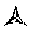 Genetic disorders
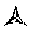 Musculoskelatal & Joint diseases/disorders
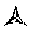 Connective Tissue diseases/disorders
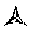 Rheumatological diseases/disorders
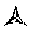 Oral diseases/disorders
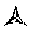 Gynecological & Obstetric disease/disorders
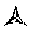 Hearing loss & Deafness
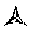 Neurologic & Neurodegenerative diseases/disorders
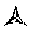 Injuries/Wounds and Wound Healing
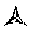 Inflammation
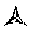 Fibrosis and Necrosis
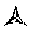 Immune Response
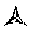 Infectious diseases (including bacterial, viral, and other pathogen infections)

It is also within the scope of the present invention that methods and kits are provided that diagnose and treat or prevent disorders associated with Gsk3b as exemplified by the list herein below:

### Disorders Associated with Gsk3b:

### Aberrant Protein Expression/Activity

- 1): Type 2 Diabetes and Insulin Resistance (15, 18, 22)
- 2): Obesity (5)
- 3): Artherosclerosis (17)
- 4): Cardiac hypertrophy (2, 4, 23)
- 5): Myocardial ischemia/reperfusion injury (1, 7, 19)
- 6): Osteoperosis (6, 14)
- 7): Myotonic dystrophy (3)
- 8): Leukemia (24, 42, 46)
- 9): Oral cancer (16, 27, 28)
- 10): Pancreatic cancer (20, 27, 34, 35, 39, 44)
- 11): Renal cell carcinoma (RCC) (34, 51)
- 12): Liver cancer (21, 27, 34)
- 13): Stomach cancer (27, 34)
- 14): Colorectal cancer (27, 34, 38, 41)
- 15): Small intestine cancer (27)
- 16): Salivary gland cancer (27)
- 17): Larynx cancer (27)
- 18): Esophageal cancer (27)
- 19): Skin cancer -cutaneous SCC, melanoma, and basal cell carcinoma (25, 26)
- 20): Breast cancer (29, 30,31, 32, 33)
- 21): Lung cancer (36, 37)
- 22): Nasopharyngeal cancer (40)
- 23): Myeloma (43)
- 24): Ovarian cancer (45)
- 25): Thyroid cancer (47)
- 26): Glioma (48)
- 27): Gastrointestinal cancer (49)
- 28): Prostate cancer (50)
- 29): Neuroblastoma (68)
- 30): Alzheimer's disease (60, 61, 62)
- 31): Huntington's disease (69)
- 32): Bipolar disorder (63)
- 33): Depression (63)
- 34): Schizophrenia (63)
- 35): Inflammation (67)
- 36): Septic Shock (67)

### Genetic variations:

- 1): Alzheimer's Disease (56, 57, 58, 59, 64)
- 2): Parkinosn's Disease (65, 66)
- 3): Familial Frontotemporal Dementia (FTD) (64).
- 4): Major Depressive Disorder (MDD) (8, 9 10).
- 5): Bipolar Disroder (11, 12, 13).
- 6): Polycystic Ovarian Syndrome (PCOS) (52).
- 7): Lung cancer (53).
- 8): Oral Squamos Cell Carcinoma (OSCC) (16).
- 9): Bone disease in Multiple Myeloma (54)
- 10): Multiple Sclerosis (55).

More specifically, the following disorders can be diagnosed and/or prevented by the methods and kits or means provided by the present invention:

### Diseases associated with cell differentiation:

Cancer - Abnormal differentiation or de-differentiation of cells is often seen in tumors. Cancer is often diagnosed too late. Very difficult to treat, and treatment regimens are harsh. Affects many people, comes in many different types.

Myelodysplastic Syndrome (MDS) - A very rare disease (orphan drug) caused by exposure to radiation or toxins, characterized by impaired differentiation of blood cells. MDS Affects mostly people over 50 or 60. Causes severe anemia, and often leads to blood cancer (AML). Important to distinguish it in diagnosis from other anemias; blood counts and bone marrow counts are used in diagnosis.

Fibrodysplasia ossificans progressiva (FOP) - A very rare disease (orphan drug) in which muscle tissue turns into bone. The disease appears in early childhood, causes severe handicap. Injuries or minor trauma causes muscle to turn into bone throughout childhood. It is diagnosed by x-rays or other scans. Biopsies make the condition worse. Not many good treatment options are available. The disease is caused by a dominant mutation.

### Diseases associated with protein synthesis:

Cancer - Abnormal protein synthesis is often seen in cancer. Cancer is often diagnosed to late. Very difficult to treat, and treatment regimens are harsh. Affects many people, comes in many different types.

### Diseases associated with cytoskeleton/microtubules:

Liver disease/cirrhosis - Many different types of liver disease are characterized by changes in the microtubules and cytoskeleton. Scarring and damage to the liver is often irreversible. Disease is often diagnosed by blood tests (liver enzymes) and/or biopsy. Liver disease is somewhat hard to treat and can often lead cancer.

Parkinson's disease - Debilitating neurodegenerative condition. Some research shows that changes proteins (Parkin) which interact with the microtubules play a role in some cases. Parkinson's is impossible to treat, most treatment options only slow down the disease, at best. Diagnosis is done by MRI and clinical manifestations (not a good candidate for us, because you can't do brain biopsies).

Alzheimer's disease - Debilitating neurodegenerative condition. Some research shows that changes proteins (Tau) which interact with microtubules play a role in some cases. Alzheimer's is impossible to treat, most treatment options only slow down the disease, at best. Diagnosis is done by MRI and clinical manifestations (not a good candidate for us, because you can't do brain biopsies).

### Diseases associated with proliferation/apoptosis:

Cancer - caused by increased proliferation and decreased apoptosis; also abnormal differentiation or de-differentiation of cells is often seen in tumors, and abnormal protein synthesis is often seen in cancer. Cancer is often diagnosed too late. Very difficult to treat, and treatment regimens are harsh. Affects many people, comes in many different types.

### Diseases associated with proliferation (rare diseases/orphan drugs):

Synovial osteochondromatosis (SOC) - Rare disease (orphan drug) of the joints that affects adults, usually beginning between age 20-40. The disease is caused by proliferation or changes in the synovial fluid of the joints. The diagnosis of the disease is done by MRI or other imaging. The treatment is done by surgery. The molecular mechanisms are not understood.

Lymphangiomyomatosis (LAM) - Rare disease (orphan drug) involving benign proliferation of cells in the lungs. Almost exclusively affects women of child-bearing age. Treatment is often hormonal therapy. The disease is sometimes not diagnosed until late stages, because of misdiagnosis in the early stages.

### Diseases associated with apoptosis (common):

AIDS - One of the biggest killers in the world. It occurs as a result of HIV infection, and it causes apoptosis of CD4+ T cells. The HIV disease is very difficult to treat.

Mono (infectious mononucleosis) - A common viral infection which causes T cells to induce apoptosis of infected B cells. Most patients recover on their own within a few weeks, but there is no way to speed this up or alleviate symptoms. The disease spreads very easily by contact with saliva or other body fluids.

### Diseases associated with apoptosis (rare diseases/orphan drugs):

Whipple's disease - a rare (orphan drug), chronic bacterial infection that can be deadly if untreated. Usually affects adult men, more common in farmers/people who work the land. Causes apoptosis of macrophages, and other T-cells attack the intestinal tracts. There are good diagnostic tools available (PCR-based blood test or histology), but often takes a while to diagnose, because there are symptoms affecting several organs but they take years to develop/progress. The conventional treatment is by long-term antibiotics.

Autoimmune lymphoproliferative syndrome (ALPS) - A very rare disease (orphan drug) in which there is a defect in lymphocyte (T-cells and B-cells) apoptotic pathways (usually Fas), causing increased lymphocyte survival. This is non-malignant proliferation, but can lead to autoimmune disorders, secondary cancers. Treatment is harsh, similar to that of autoimmune disorders. Diagnosis is by blood test that has several criteria.

Systemic capillary leak syndrome (SCLS) - A very rare disease (orphan drug) involving recurring episodes which fix themselves, but can also be fatal. Very difficult to diagnose, because often initially resembles a cold or flu. The disease is caused by a sudden leakage of blood plasma into body cavities and muscles, which causes a sharp drop in blood pressure and pain/ swelling of hands, feet, and other organs. After a while, the fluid returns to the blood vessels. Not well understood, though some studies show apoptosis is involved. The disease is difficult to diagnose. Treatment involves management by steroids (cannot be cured, though it can go into remission for many years).

### Diseases associated with migration and/or proliferation (autoimmune):

Atheroscelrosis - A very common risk factor in the population for many fatal conditions (heart attack, stroke). The disease is caused by an immune response to injury, cholesterol, and plaque buildup in the arteries. Diagnostic techniques, such as stress tests can detect narrowing of the arteries (indicating severe build-up); earlier diagnosis is done by blood test and/or imaging but are not widespread. Prevention (healthy lifestyle) is the best cure. Other common treatments use oral medication or surgery.

Type I Diabetes - Diabetes is one of the most common conditions. Type I is caused when the body's immune system attacks the pancreatic cells which produce insulin. Treatment is done by management of insulin levels, by insulin injections and diet regulation.

Rheumatoid arthritis - Autoimmune disorder mainly affecting the joints, appearing often in people aaged 40-50. Diagnosed by clinical symptoms, blood tests, and imaging. Treatment is harsh, by steroids and/or immunosuppressants, among others.

Lupus (SLE) (and other autoimmune disorders) - A rare multi-systemic autoimmune disorder, affecting adults, and more common in women, usually appearing between age 20 and 40. Diagnosed by clinical symptoms, blood tests, and imaging. Treatment is harsh, by steroids and/or immunosuppressants, among others.

Sjogren's Syndrome - A rare autoimmune disorder in which the body's immune system attacks exocrine glands (tears, saliva, etc); occurs in a dults, but is much more common in women. This autoimmune disorder can be diagnosed by blood tests, tear tests, and/or gland imaging. Treatment is aimed at managing symptoms.

Scleroderma - A rare multi-systemic autoimmune disorder, affecting adults. Diagnosed by clinical symptoms, blood tests, and imaging. Treatment is harsh, by steroids and/or immunosuppressants, among others; better treatment options are needed.

Inflammatory Bowel Disease (Crohn's disease and ulcerative colitis) - These rare gastrointestinal disorders and are characterized by migration of T-cells to the gut and they attack the gastrointestinal tract. These disorders are usually diagnosed by colonoscopy and blood tests for inflammatory markers. Treatment is harsh, by steroids and/or immunosuppressants, among others.

Multiple Sclerosis - A rare disease in which the body's immune system attacks the neurological system, causing a neuro-muscular "attacks". It usually occurs in adults, and most patients eventually lose the ability to walk, among other motor functions. Diagnosis is done by biopsy, MRI, and/or electrophysiological tests. There is no cure, but several treatment options can be helpful, though better options are needed.

### Diseases associated with migration and proliferation (non-autoimmune):

Langerhans cell histiocytosis - a very rare disease that usually occurs in children between ages 1-15, and even less frequently in adults. The disease involves proliferation and migration of different blood cell types and subsequent migration/invasion in other organs. The diagnosis of this disease is usually done by biopsy. Treatment is very aggressive (steroids, immunosuppressants, chemotherapy, radiation).

Examples of antibodies, antigens or biomarkers used in the diagnostic kit of the present invention may include:

### 1. GSK-3β Activation State:

### 1. GSK-3β Antibodies:

- Total GSK-3β
- p-GSK-3β Ser 9 phosphorylation - indicates GSK-3β inactivation
- p-GSK-3β Tyr216 phosphorylation - indicates GSK-3β activation
- p-GSK-3β Thr43 phosphorylation - priming phosphorylation for Ser9 phosphorylation
- p-GSK-3β Thr390 phosphorylation - indicates GSK-3β inactivation (Human only)

### 2. Proteins Upstream of GSK-3β:

- PKC - directly phosphorylates (and inhibits) GSK-3β.
- Akt - part of PI-3K signaling cascade protein which phosphorylates (and inhibits) GSK-3β.
- Erk - Map kinase signaling cascade protein that directly phosphorylates (and inhibits) GSK-3β.
- P38 MAPK - Map kinase signaling cascade protein that directly phosphorylates(and inhibits) GSK-3β
- Dvl - a protein upstream of GSK-3β in the Wnt pathway, and an indicator of Wnt pathway activation

### 3. Monitoring of Other Cellular Processes:

- Markers for apoptosis (Fas, Bcl family, Cyctochrome C, caspases)
- Markers for immune activity (Nf-kB, CD25)
- Markers for cell proliferation (Cyclin D1, PCNA, p27)

**2. Table 1: GSK-3β Substrates and downstream products:**

| | **Name** | **References** | **Function** | **Downstream Targets** |
|---|---|---|---|---|
| **1** | αNAC | 9 | translation regulation, and bone development transcription factor | osteocalcin |
| **2** | β-catenin | 8 | wnt signaling, cytoskeleton | c-myc, c-jun, fosL1, cyclin d1, pparδ, mmp7, mitf, survivin |
| **3** | δ-catenin (delta catenin) | 60 | cell adhesion | E-cadherin, Rac-1 |
| **4** | 5-HT(1B) | 46 | serotonin receptor | No known protein products (affects cAMP) |
| **5** | AcetylCoA carboxylase | 7 | fatty acid metabolism | No protein products (affects fatty acids) |
| **6** | Amyloid precursor protein (APP) | 82 | synaptic formation | Aβ protein (brain) |
| **7** | AP-1 (Jun proteins) | 2 | transcription factor | Cyclin D, FasL |
| **8** | APC | 12 | wnt signaling | c-myc, c-jun, fosL1, cyclin d1, pparδ, mmp7, mitf, survivin |
| **9** | Astrin | 97 | spindle formation during mitosis | separase |
| **10** | Ataxin-3 | 47 | proteasome mediated protein degradation, | No known protein products (has no specific substrates) |
| **11** | ATP citrate lyase | 7 | fatty acid synthesis | No protein products (affects fatty acids) |
| **12** | Axin | 8 | wnt signaling | c-myc, c-jun, fosL1, cyclin d1, pparδ, mmp7, mitf, survivin |
| **13** | Bax | 48 | apoptosis | Casp-9, Casp-3 |
| **14** | BCLAF1 | 94 | apoptosis - transcription factor | Unknown products |
| **15** | BMAL1 (ARNTL) | 50 | circadian rythym | PER1 |
| **16** | C/EBP | 20 | transcription factor | IL-6, E2f, Myc |
| **17** | CABYR | 51 | sperm structural protein | No protein product (structural protein) |
| **18** | Cdc25A | 74 | cell cycle - kinase | CDC2 (CDK1) |
| **19** | CdGAP | 103 | cell migration | actin |
| **20** | CDX2 | 5 | intestine transcription factor | lactase, MUC2, LI-cadherin, Claudin-2, p21 |
| **21** | CIITA | 56 | MHC class II expression - transcription factor | MHC class II |
| **22** | CLASP2 | 90 | cell migration, chromosome alignment | No protein products (sits on cytoskeleton) |
| **23** | CLOCK | 57 | circadian rhythm - transcription factor | PER1, PER2 |
| **24** | CREB | 10 | transcription factor | Fos |
| **25** | CRMP2 (DRP2)/CRMP4 | 35 | neuron development | No protein products (affects axon growth directly) |
| **26** | CRY2 | 58 | circadian rhythm | PER1, PER2 |
| **27** | Cyclin D1 | 40 | cell cycle | E2F, Rb |
| **28** | Cyclin E | 42 | cell cycle | E2F, Rb |
| **29** | DF3 (MUC1) | 18 | surface glycoprotein, binds pathogens, cell signalling | Ras, p53, b-catenin, ERK, NF-kB |
| **30** | Doublecortin | 61 | neurogenesis | tubulin? |
| **31** | DSCR1 | 71 | calcineurin-mediated signaling pathway | NFAT |
| **32** | Dynamin-1 | 63 | neuron endocytosis | No protein product (endocytosis protein) |
| **33** | Dynamin-like protein | 43 | vesicle formation | No protein product (affects mitrochondrial fission) |
| **34** | Dystrophin | 45 | cytoskeleton and extracellular matrix | No protein products (it's an anchoring protein). |
| **35** | E-Cadherin | 54 | call adhesion protein | No protein product (adhesion protein) |
| **36** | E2F1 | 64 | cell cycle - transcription factor | Casp-9, Cyclin E, c-Myc |
| **37** | eIF-2B | 3 | translation factor | elF2-GTP |
| **38** | ER-α | 65 | estrogen receptor - transcription factor | cathepsin D, cyclin D1, c-Myc, progesterone receptor |
| **39** | FBX4 | 77 | protein degradation | cyclin D1 |
| **40** | FRAT1 | 67 | Wnt signalling | b-catenin |
| **41** | GATA4 | 102 | embryogenesis, myocardial differentiation - transcription factor | ANF, α-myosin heavy chain, cTnC |
| **42** | GCM1 | 86 | placenta development - transcription factor | placental growth factor (PGF), syncytin 1 |
| **43** | gephyrin | 69 | CNS scaffold protein | No protein products (scaffold protein) |
| **44** | Gli (Ci homolog) | 36 | embryonic development - transcription factor | PTCH, HNF3β, PDGFRα, MYF5, Wnt, BMP, HIP |
| **45** | Glucocorticoid receptor (GR) | 30 | cortisol/glucocrticoid binding - cytokine expression regulation | GILZ, IGFBP1, IRF8 |
| **46** | Glycogen Synthase | 6 | glycogenesis | No protein products (affects glycogen) |
| **47** | H1B | 70 | histone | No protein products (histone) |
| **48** | HDAC4 | 62 | histone deacetylase | runx2, p21, HIF1a |
| **49** | HIF-1 | 73 | blood vessel formation - hypoxia signaling | VEGF |
| **50** | hnRNP | 104 | mRNA processing | No protein products (affects splicing) |
| **51** | HSF-1 | 15 | stress response - ranscription factor | Hsp70, Hsp72 |
| **53** | Insulin receptor substrate-1 (IRS-1) | 19 | insulin signaling | PDK1/2 |
| **54** | Kinesin light chain (KLC) | 33 | intracellular transport | No protein product (transport protein) |
| **55** | KLF5 | 72 | transcription factor | FGF-BP, PDGF, cyclin D1, PPARg. |
| **56** | LPCAT1 | 37 | lung surfactant compound production | No protein products (makes biochemicals in surfactant) |
| **57** | LRP6 | 52 | Wnt signalling | b-catenin |
| **58** | LU (BCAM) | 75 | red blood cell receptor | Rap-1 |
| **59** | MAP1B | 99 | neuron development | Tubulin? |
| **60** | MAP2 | 25 | neuron development | Tubulin? |
| **61** | MARK2 (Par-1) | 76 | epithelial morphogenesis | Tau, MAP2c, MAP4 |
| **62** | Mash1 | Mentioned in a review (107), but no experimental data found | neurogenesis | c-myc, c-jun, fosL1, cyclin d1, pparδ, mmp7, mitf, survivin |
| **63** | MCL1 | 66 | apoptosis pathways | Casp-9, Casp-3 |
| **64** | Mdm2 | 38 | negatively regulates p53 | Rb,p53 |
| **65** | MEF2D | 78 | Neuron & muscle development - transcription factor | c-Jun |
| **66** | MITF | 32 | melanocyte and osteoclast development - transcription factor | tyrosinase, tyrosine-related-protein-1 |
| **67** | Myb | 55 | transcription factor | Cyclin B, Myc, CXCR4 |
| **68** | Myc (c-Myc, L-myc) | 4 | transcription factor | eIF2a, p27, p53, CCR6 (L-myc) |
| **69** | | 26 | nerve myelination | No protein products (affects brain cell myelination) |
| **70** | myocardin | 79 | cardiac and smooth muscle development | atrial natriuretic factor (ANF), calponin-h1 |
| **71** | Neural cell-adhesion Protein (NCAM) | 29 | surface glycoprotein - many cell types | p59fyn, PI-3k , PLCγ |
| **72** | NeuroD | Mentioned in a review (107), but no experimental data found | nervous system differentiation, pancreas development | insulin, CDKN1A, secretin |
| **73** | Neurofilament (NFH) | 31 | neuron cytoskeleton | No protein products (affects axon growth directly) |
| **74** | neurogenin 2 | 14 | motor neuron differentiation | NeuroM, NeuroD, and β-III-tubulin |
| **75** | NF-κB (p65 and p105) | 34 | immune response - transcription factor | Bcl2, IFN, TNFa, IL-12, IL-6, IL-10, IL-8, GM_CSF, CDK4 |
| **76** | NFAT | 17 | immune response - transcription factor | IL-2, IL-4, IL-5, IL-10, IFN-γ, GM-CSF, TNF-α, CDK4 |
| **77** | NGF receptor | 89 | neuron growth and survival | Ras, Cdc42, MAPK, PI3-K, PLCgamma, Nf-kB |
| **78** | NIFK | 83 | nucleolar protein of unknown function | No known protein products |
| **79** | Ninein | 16 | centrosome function (mitosis?) | Not enough known |
| **80** | Notch | Mentioned in a review (107), but no experimental data found | cell differentiation | PTCRa, HES1 |
| **81** | Nrf-2 | 28 | oxidative stress pathways | NQO1, HO-1, glutathione S-transferase |
| **82** | Nucleoporin p62 | Mentioned in a review (107), but no experimental data found | nuclear envelope protein | No protein products (does RNA transport at nuclear envelope |
| **83** | p120 catenin | 59 | cell adhesion | E-cadherin, Nf-kB |
| **84** | p21 | 39 | cell cycle | Cyclin D, Cyclin E |
| **85** | p53 | 41 | cell cycle and others | PTEN, p21, Fas, PCNA |
| **86** | PAX3 | 85 | early skeletal muscle development - transcription factor | MyoD |
| **87** | Paxillin (PXN) | 106 | Cell migration | Actin? |
| **88** | PDX1 | 68 | Pancreas - transcription factor | insulin, Glut2, glucokinase |
| **89** | PKA (RII subunit) | 21 | metabolism regulation | Rap1, Bad |
| **90** | PKCε | 87 | PKC pathway | VDAC1, BAD |
| **91** | Presinilin 1 (PS-1) | 11 | neurotransmitter release | CSL (notch signaling), Aβ protein (brain) |
| **92** | Protein phosphatase 1 | 23 | metabolism | Axin |
| **93** | Protein phosphatase inhibitor-2 | 92 | metabolism | Erk, Akt |
| **94** | PSF | 88 | splicing factor | CD45R0 isoform |
| **95** | PTEN | 22 | negative regulator of the PI3K/Akt signaling pathway | Akt |
| **96** | Pyruvate dehydrogenase | 27 | glycolysis | No protein products (affects sugars/fatty acids) |
| **97** | RASSF1A | 91 | Apoptosis, others | Bax, Cyclin D1 |
| **98** | ReIB | 24 | NfkB transcription complex | NFATc, IFNb, TNFb, IL6 |
| **99** | Rev-erb-α (NR1D1) | 84 | circadian rhythm | BMAL1 |
| **100** | SIK | 105 | cardiac and skeletal muscle development | CRTC1-3, HDAC-5 |
| **101** | Smad1 | 49 | bone morphogenesis | Id |
| **102** | Snail1 | 95 | Embryogenesis - transcription factor | E-cadherin |
| **103** | SREBP-1 | 98 | cholesterol & lipid metabolism, adipocyte differentiation | HMGS, LDLr, FPPS |
| **104** | SUFU | 80 | hedgehog signaling pathway | PTCH, Wnt, BMP, HIP |
| **105** | Synphilin-1 | 96 | alpha-synuclein interacting protein | a-synuclein |
| **106** | Tankyrase | 101 | chormosome segragation in mitosis, telomere maintenance | axin, TERF1 |
| **107** | Tau | 1 | neuron development | Tubulin |
| **108** | TCF | Mentioned in a review (107), but no experimental data found | transcription factor | c-Myc, c-Jun, Cyclin D, PPARg, MITF, Survivin |
| **109** | Telokin (KRP, smMLCK) | 44 | muscle filament stabilization | Myosin light chain |
| **111** | Tip60 | 100 | chromatin remodeling, apoptosis | PUMA, p53, chk2 |
| **112** | Topollα | 13 | Control of aberrant topological states of DNA | No protein products (works directly on DNA) |
| **113** | TSC2 | 53 | mTOR signaling pathway | S6K1, 4E-BP1 |
| **114** | VHL | 81 | oxygen-sensing pathway, possibly also interacts with microtubule | HIF1A |
| **115** | ZBED3 | 93 | Binds axin, Unknown function | β-catenin |

### EXAMPLES

### Example 1: GVHD Mouse Model

### Goal:

To demonstrate that the levels of GSK-3β and proteins from related pathways, can be used as markers for pathological conditions associated with T-cell proliferation, such as GVHD diagnosis, at an early disease stage.

### Materials and methods

**Mouse Model:** Eight- to 10-week-old male C57BL/6 and Balb/c mice were purchased from the Harlan Animal Farm. The animals were maintained in a pathogen-free animal facility at the Hadassah-Hebrew University Medical School, Jerusalem, Israel. Animals were treated according to the standards of the Animal Ethics Committee, Hebrew University Medical School Animal Care Facilities. Mice were harvested and samples were collected from blood and spleen on days 0, 1, 3, 4, 5, & 6 post-transplant.

**Splenocyte Collection:** Mice were sacrificed at the indicated time points and spleens were harvested. Splenocytes were produced by passing spleens through a mesh screen in PBS, and processed with erythrocyte lysis buffer, and washed with cold PBS. Donor splenocytes were processed the same way.

**Mouse Splenocyte Transplant:** Mice are divided into 3 groups (Untreated, Syngeneic and Allogeneic) per time point with 6-12 mice per group. The untreated group mice (without irradiation) were injected with plain medium. The syngeneic group irradiated (600 rads) Balb/c mice were injected with splenocytes (30 x 10^6) from matched donor Balb/c mice. The allogeneic group irradiated (600 rads) Balb/c mice were injected with splenocytes (30 x 10^6) from unmatched donor C57B1/6 mice. An additional 2-3 mice in each group were injected wit 2.0 mg/kg LiCl (dissolved 1:2 LiCl:PBS), once a day from day 1 to day 5 post-transplant, sacrificed at days 3 and 5.

**FACS analysis of Apoptosis:** Splenocytes were washed twice with cold FACS buffer (1x PBS/0.5% BSA/0.02% sodium azide), and incubated on ice for 30-45 min with APC-conjugated anti-CD3. FITC-conjugated anti-annexin-V mAb was used as a marker of early stages of apoptosis. To differentiate apoptotic cells from necrotic ones, splenocytes were co-stained with PI and annexin-V-FITC in a PI kit (MBL), according to the manufacturer's protocol. Flow cytometry was performed on a FACScan, and data were analyzed using CellQuest software (Becton Dickinson). A total of 5x105 CD3+ cells were counted for each sample.

**FACS analysis of Proliferation:** Splenocyte were washed with cold PBS, and fixed with 95% ethanol overnight at 20°C. On the following day, splenocytes were washed twice with FACS buffer (PBS supplemented with 0.5% BSA and 0.01% sodium azide), and incubated on ice for 30-45 min with APC-conjugated anti-CD3. Before flow cytometry, splenocytes were resuspended in PBS containing 5 mg/ml propidium iodide (PI) and 10 µg/ml RNase A (Sigma-Aldrich). Fluorescence-labeled cells were analyzed on a FACScan, using a CellQuest software package (Becton Dickinson).

**Western Blot**: Splenocytes were lysed in NP-40 lysis buffer (0.5% Nonidet P-40, 50 mmol/L Tris-HCl [pH 8.0], 100 mmol/L NaCl, 1 mmol/L phenylmethylsulfonyl fluoride, 1 mmol/L sodium orthovanadate, 10 µg/ml leupeptin, and 10 µg/ml aprotinin) for 20 to 30 minutes on ice. Protein concentration was determined by Bradford reagent (Bio-Rad) Lysates were mixed with 2x Laemmli sample buffer at 1:1 ratio, heated for 5 minutes at 95°C, and equal amounts of protein were loaded onto 10% SDS-polyacrylamide gel electrophoresis (PAGE). Following electrophoresis, gels were blotted onto nitrocellulose membranes (Bio-Rad), blocked with 5% milk/PBS, and probed overnight with primary Abs. After extensive washing, blots were incubated with horseradish peroxidase-conjugated matching secondary Abs (Bio-Rad), and developed with enhanced chemiluminescent substrate (Sigma-Aldrich) before exposure to X-ray film (Fuji Film). Membranes were subsequently incubated with α-tubulin or GAPDH to ensure equal protein concentrations in each sample. Films were scanned and protein bands were quantitated using ImageJ software (NIH).

**MLR:** Splenocytes were harvested from untreated Balb/c and C57BL/6 mice. C57BL/6 cells were incubated in a flat bottom 96 well plate for 96 h with either irradiated (1500 rads) or not irradiated Balb/c cells as indicated, at the indicated concentrations. XTT reagent for proliferation assay was added 24 h before reading. Plate was read at 620 nM.

**Antibodies and Reagents:** Anti-mouse CD3 mAb were purchased from R&D Systems. For Western blot analysis, anti-Gsk-3β Ab, anti-p-Gsk-3β (ser9) Ab, anti-p-PKC Ab, and and anti-α-tubulin Ab were purchased from Cell Signaling. Anti-mouse GAPDH Ab were purchased from Sigma-Aldrich. XTT kit, PBS and cell medium were purchased from Biological Industries (Israel). All other reagents were purchased from Sigma-Aldrich unless otherwise indicated. Disposables were purchased from Corning.

### Experimental procedure:

Samples were collected from blood and spleen on days 0, 1, 3, 4, 5, & 6 post-transplant, mainly from the following 3 groups (at least 3 mice per group):
- **Group 1-Untreated/control (Con):** injected with plain medium (no irradiation).
- **Group 2- Syngeneic (Syn):** Balb/c mice injected with a splenocyte transplant from matched donor Balb/c mice after irradiation.
- **Group 3- Allogeneic (Allo):** Balb/c mice injected with a splenocyte transplant from unmatched donor (i.e.C57B1/6 mice) after irradiation.

### Results

The results obtained show that GVHD mice have significantly lower levels of p-GSK-3β (phosphorylated in Ser-9) and total GSK-3β than both syngeneic and untreated control mice. This effect has been observed over time and is most significant on days 4-5 post transplant, both in the spleen and the blood samples. The main significance of these results is that the present invention provides for the first time a tool to diagnose and predict pathological conditions of T-cell proliferation, such as the diagnosis of GVHD effects in a subject, before emergence of the symptoms of the disease.

### 1. Spleen results

### a. Total GSK-3β and p-GSK-3β expression levels

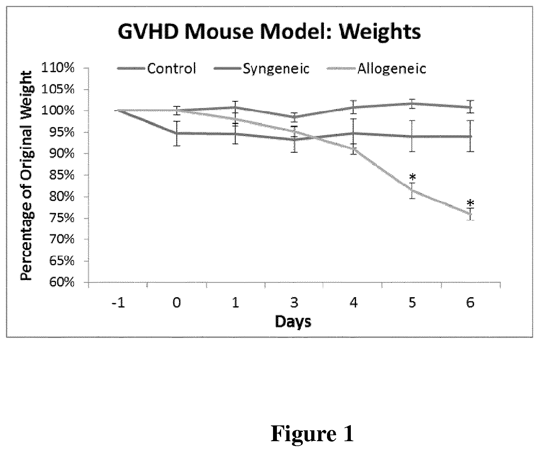

The allogeneic mice developed GVHD symptoms by day 6 post-transplant. The symptoms of GVHD seen in the allogeneic mice included, but were not limited to, ruffled fur, hunched posture, closed eyes, reduced activity, diarrhea and weight loss. As shown in **Figure 1****,** the weight loss in the allogeneic mice is significantly increased on days 5-6, in correlation with the disease progression.

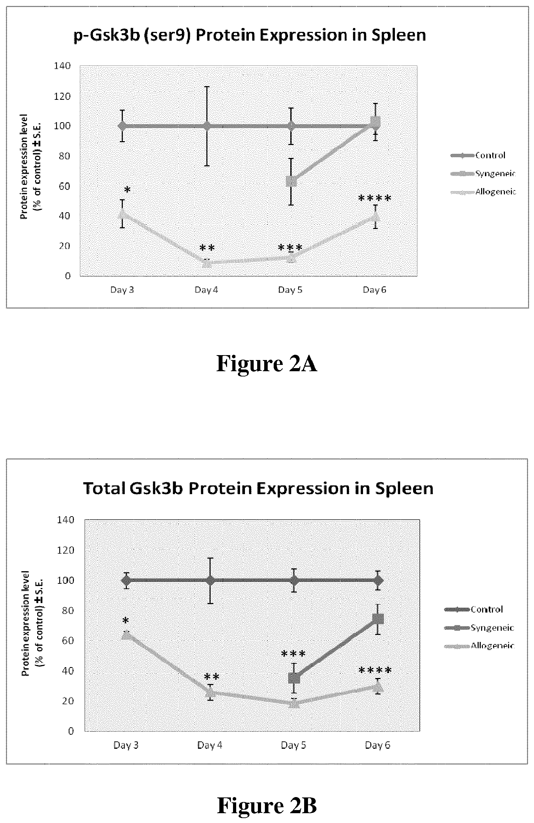

Reference is now made to **Figure 2****,** showing spleen results of p-GSK-3β and total GSK-3β expression.

The results have been normalized such that the values for the untreated control mice were set as 100%. Each parameter was compared to the values of the untreated mice.

As shown in **Figure 2A and 2B****,** the mice in the untreated control group are healthy mice. In the syngeneic group, short-term effects of irradiation and full recovery were observed with no GVHD symptoms. However the allogeneic mice showed short term effects of irradiation as well as subsequent GVHD development.

More particularly, a prolonged decrease in the level of p-GSK-3β and total GSK-3β over time was exclusively detected in the spleen of the allogeneic group mice. It should be mentioned that in the Syngeneic group, the splenocyte population has not recovered enough to collect cells, until day 5.

**Figure 2A****,** demonstrates that the expression level of p-GSK-3β in the allogeneic group, on day 3 (* Con & Allo p<0.02) and day 4 (** Con & Allo p<0.05), before appearance of GVHD symptoms, was significantly decreased relative to the control. On day 5 (*** Con & Allo p<0.01; *** Syn & Allo p<0.01; Con & Syn = no significant difference) once severe symptoms have appeared in the mice, the level of phosphorylated GSK-3β in the allogeneic mice was maintained at a relatively lower level than the other groups, and on day 6 (**** Con & Allo p<0.01; **** Syn & Allo p<0.01; Con & Syn = no significant difference) the level of p-GSK-3β in the syngeneic and allogeneic mice appears to approach almost normal.

As shown in **Figure 2B****,** the detection of the expression level of total GSK-3β in the allogeneic group, showed the same tendency and early effect, before appearance of the symptoms. On days 3 and 4 (* Con & Allo p<0.05), before appearance of GVHD symptoms, a significant decrease was observed in the expression level of total GSK-3β relative to the control. On day 5 (*** Con & Allo p<0.01; Syn & Allo= no significant difference; Con & Syn p<0.01) once severe symptoms have appeared in the mice, the level of total GSK-3β in the allogeneic mice was maintained at a lower level than the other groups, and on day 6 (**** Con & Allo p<0.01; ****Syn & Allo p<0.01; Con & Syn = no significant difference) the level of in the allogeneic group maintained at a lower level while the level of total GSK-3β in the syngeneic mice appears to approach normal.

To summarise the results described hereinabove, there is a prolonged decrease of total GSK-3β and p-GSK-3β over time in the spleen exclusively seen in the allogeneic group. The fact that in the syngeneic group, the splenocyte population has not been recovered enough to collect cells until day 5, while in the allogeneic mice there were enough splenocytes already on day 3, may be a result of the immune response characteristic of GVHD.

Once severe symptoms have appeared (days 5-6), levels of p-GSK-3β and total GSK-3β in allogeneic mice maintained lower than other groups. It is noted that the fact that in the syngeneic mice, the expression level of GSK-3β appears to approach almost normal by day 6, may reflect the effect of recovery from irradiation.

In the allogeneic group, p-GSK-3β and total GSK-3β decrease before the appearance of clinical symptoms (symptoms begin on day 5) and maintain lower than other groups throughout the disease course.

### b. PKC expression level

Protein Kinase C (PKC) is upstream of GSK-3β in several pathways. Particularly, it is capable of phosphorylating GSK-3β directly. It is shown by the results of the present invention that activated PKC levels are significantly lower in splenocytes of both the syngeneic and allogeneic mice.

It is noted that the results described herewith have been normalized such that values for the control mice were set at 100%. Each parameter was compared to the value of untreated (control) mice independently of other parameters.

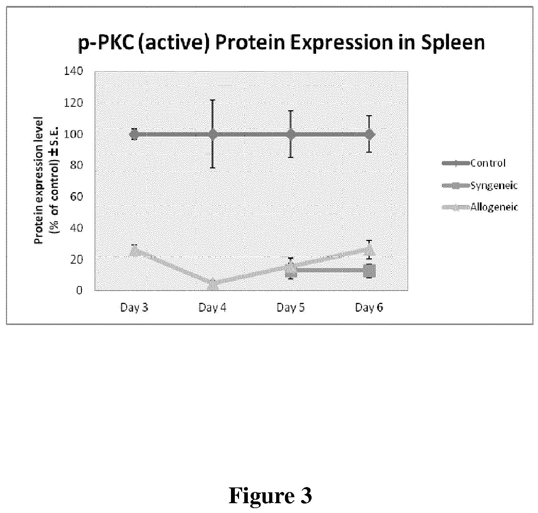

As shown in **Figure 3**, a decrease in PKC expression is shown in both the syngeneic and allogeneic groups. Thus it can be concluded that the decrease in PKC expression relative to the control mice could be due to irradiation or transplantation.

### c. T-cell (CD3⁺) proliferation level

Total T cells (CD3+) were collected from spleens of mice of the 3 groups of the GVHD model as described above. Cells were stained for CD3 and counted by Flow Cytometry.

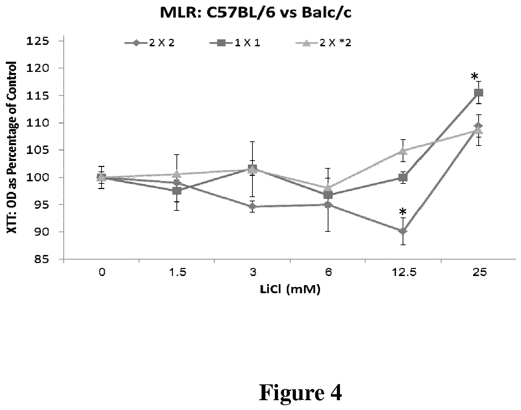

Reference is now made to **Figure 4**, showing percentage of CD3+ cells out of total splenocytes. As shown in this figure, on day 5 the allogeneic mice have significantly more CD3+ cells than the control and syngeneic mice (**t-test p<0.01). On the other hand, the syngeneic mice have less CD3+ cells than control mice (*t-test p<0.05).

In order to explore the effect of GSK-3β activation state on T- cell proliferation, the GVHD model mice were treated with Lithium chloride (LiCl). Lithium chloride (LiCl) is known to affect GSK-3β activation state by regulating its phosphorylation state. Lithium chloride (LiCl) treatments were given to a separate group of allogeneic mice. The data obtained shows that the amount of T-cells increased after LiCl treatments on day 3 post-transplant. LiCl is a known inhibitor of GSK-3β, suggesting that the increase in T cells is due to increased inhibition of the GSK-3β.

Thus, according to certain embodiments of the invention, it is concluded that by monitoring and/or affecting GSK-3β expression and/or activation state, and/or related proteins thereof, early diagnosis and prevention of GVHD is achieved.

### d. Mixed Lymphocyte Reaction

Splenocytes were collected from spleens of C57B1/6 and Balb/c mice and cultured ex-vivo and assayed for proliferation by XTT (tetrazolium salt). The C57B1/6 and Balb/c cells were cultured ex-vivo together to mimic a GVHD reaction. The cells were cultured in 3 groups, having different cell concentrations:
- 2x2: 2x10^5 C57B1/6 and 2x10^5 balb/c cells
- 1x1: 1x10^5 C57B1/6 and 1x10^5 balb/c cells
- 2x*2: 2x10^5 C57B1/6 and 2x10^5 irradiated balb/c cells

In order to test the effect of GSK-3β activation state on cell proliferation, Lithium Chloride (a known inhibitor of GSK-3β) was added at different concentrations. As shown in Figure 4, LiCl appears to have a dose dependent effect (particularly, 12.5 mM and 25 mM) on cell proliferation. Furthermore, it can be seen that the effect of LiCl depends on the confluence (concentration) of cells. More specifically, as shown in Figure 4, the proliferation level in 12.5 mM LiCl, is significantly lower in the 2x2 group than in the control (0 mM). In LiCl concentration of 25 mM, the proliferation level is significantly higher in both the 2x2 and 1x1 groups than in the control (0 mM). As shown in **Figure 4**, LiCl had no apparent effect on proliferation at doses smaller than 12.5 mM.

### e. Cell cycle analysis

Splenocytes were collected from mice of the GVHD model groups. T cells (CD3+) were analyzed by flow cytometry (FACS). Cell Cycle analysis was performed by detecting proliferating cells in each group.

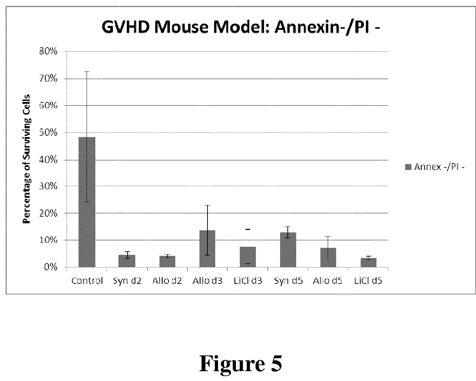

Reference is now made to **Figure 5** demonstrating apoptosis analysis. The graphic results described in the figure show surviving cells or in other words, cells that did not undergo apoptosis. The results obtained demonstrate that there is a significant increase in apoptosis in T-cells in both syngeneic and allogeneic groups as compared to controls on days 2-5. Thus significantly enhanced apoptosis is observed at early stage of GVHD. This effect can be due to irradiation and/or transplantation.

The spleen results described hereinabove demonstrate that the decrease in the total GSK-3β and the p-GSK-3β in the spleens of allogeneic mice can be detected as early as day 3, before the appearance of observable symptoms. In view of these results it is a main aspect of the present invention that total GSK-3β, p-GSK-3β and/or proteins associated or related to GSK-3β protein are utilized as markers to identify and diagnose the onset of GVHD or any other medical condition associated with cell proliferation in mice and potentially in humans.

The results disclosed by the present invention formed the basis to the development of a novel diagnostic kit that can predict the onset of GVHD or any other medical condition associated with cell proliferation in subjects. According to one embodiment, the diagnostic kit utilizes protein chip technology to monitor the changes in the GSK-3β and/or at least one related protein thereof expression levels. In a further embodiment, the diagnostic kit utilizes changes in the phosphorylation or activation state of GSK-3β and/or at least one related protein thereof to predict the onset of GVHD or any other medical condition associated with cell proliferation.

It is further demonstrated by the experiment that once the mice have developed severe GVHD (i.e. day 5), a significant increase in T cell numbers post-transplant has been seen. In addition, it is shown by the present invention that LiCl, a known GSK-3β inhibitor, affect T-cell proliferation ex-vivo and possibly in vivo. In view of these results it is herein concluded that regulation of GSK-3β has a therapeutic use in GVHD or any other medical condition associated with cell proliferation.

### 2. Blood results

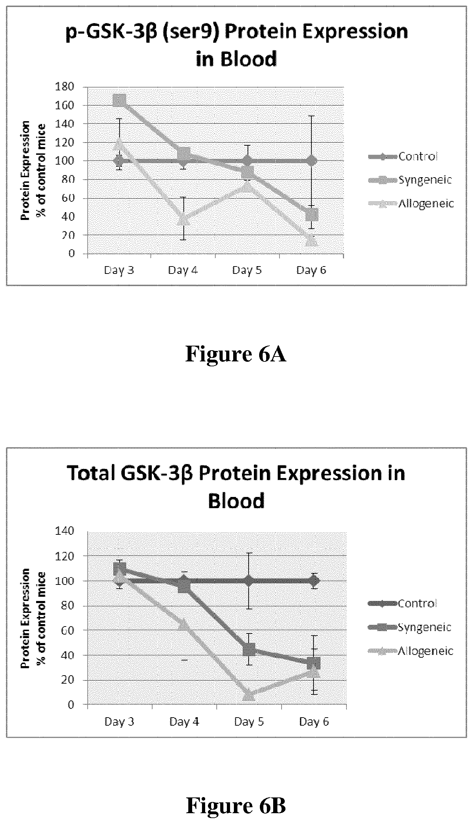

Reference is now made to **Figure 6** showing p-GSK-3β (ser 9 phosphorylated GSK-3β) (**Figure 6A**) and total GSK-3β (**Figure 6B**) protein expression in blood samples. The results have been normalized such that the values for the untreated control mice were set as 100%. Each parameter was compared to the values of the untreated mice.

The graphic results shown in **Figure 6** demonstrate that there is a prolonged decrease of total GSK-3β and p-GSK-3β over time in the blood exclusively shown in the allogeneic group.

More specifically, the results obtained show that there is a decrease in total GSK-3β and p-GSK-3β in the syngeneic mice, which seems to be later than the decrease observed in the allogeneic mice. In the allogeneic group, there is a decrease in the levels of total GSK-3β and p-GSK-3β over time, this decrease is greater in the allogeneic group versus the syngeneic group.

It can be concluded from the results obtained by the blood experiment that a decrease in total GSK-3β and the p-GSK-3β is seen in the blood in mice of the allogeneic group, which is more pronounced than both the control and syngeneic mice.

It is further indicated that the decrease is most noticeable on days 3 and 4, which are at an early stage of GVHD. It is noted that at this stage of the disease T-cell proliferation is pronounced.

Thus based on the results described herein, a diagnostic kit is provided, which enables the use of blood samples to detect the early onset of GVHD and/or any other medical disorder associated with cell proliferation. According to certain embodiments, such a kit may utilize protein chip technology to monitor the changes in the GSK-3β expression levels and/or changes in the activation state of the GSK-3β to predict the onset of GVHD and/or any other medical disorder associated with cell proliferation. According to further embodiments, such a kit may additionally include GSK-3β related proteins, and by monitoring their expression levels and/or activation (i.e. phosphorylation) state the onset of GVHD and/or any other medical disorder associated with cell proliferation is predicted.

It is indicated that the blood results in mice reflects the proliferation in the spleen, meaning that the cells in the syngeneic mice on days 3, 4, and maybe 5 are cells that existed in the periphery before the transplant. However, in the allogeneic mice, the cells from day 4 are already new cells which have emerged from the spleen.

It should be emphsised that the fact that blood samples could be obtained from mice is a good indication of the ability to collect usable blood samples from humans.

The unique data presented herewith correlates GSK-3β directly to the progression and onset of the GVHD disorder which has never been done before. The correlation between GSK-3β expression and/or activation state and GVHD progression is associated with the effect on cell proliferation. Thus by monitoring and regulating GSK-3β and /or proteins related to GSK-3β (i.e. upstream or downstream GSK-3β), medical disorders associated with cell proliferation, such as GVHD can be early detected and inhibited before the emergence of undesirable symptoms.

According to a main aspect of the invention, the changes seen in the GSK-3β or related proteins expression levels and activation state can be utilized as markers for diagnosing the GVHD and/or any other medical disorder associated with cell proliferation.

According to a further main embodiment, the early onset of GVHD is detected prior to observable clinical symptoms through monitoring changes of the GSK-3β expression levels and/or changes in the activation state of the GSK-3β protein or related proteins thereof. Using GSK-3β and/or proteins associated with GSK-3β, as markers allow the diagnosis of GVHD or any other medical disorder associated with cell proliferation prior to patients demonstrating observable symptoms and prior to the T cells attacking the host body.

It is within the scope of the present invention, that by regulating GSK-3β expression in a time-course study of an acute GVHD mouse model, GSK-3β manipulation is shown to prevent GVHD and/or any other medical disorder associated with cell proliferation onset.

### Example 2: Fingerprinting data

In another embodiment of the invention, a diagnostic kit based on a protein chip is used to diagnose GVHD and/or any other medical disorder associated with cell proliferation before the appearance of clinical symptoms. In certain embodiments the kit contains a custom-made protein chip comprising antibodies which are adapted to monitor the activation state and/ or expression level of the GSK-3β, related proteins associated with related pathways such as apoptosis markers, proliferation markers, and other biological and biochemical molecules related to GSK-3β. Such molecule candidates can be pre determined and analysed by western blot, FACS, and other conventional fingerprinting techniques.

It is herein acknowledged that every disorder has a unique fingerprint. Finding the fingerprint for GVHD and/or any other disorder associated with cell proliferation allows us to provide a diagnostic kit with a set of proteins that are specifically and uniquely correlated with a particular disorder or medical condition associated with cell proliferation, for example GVHD.

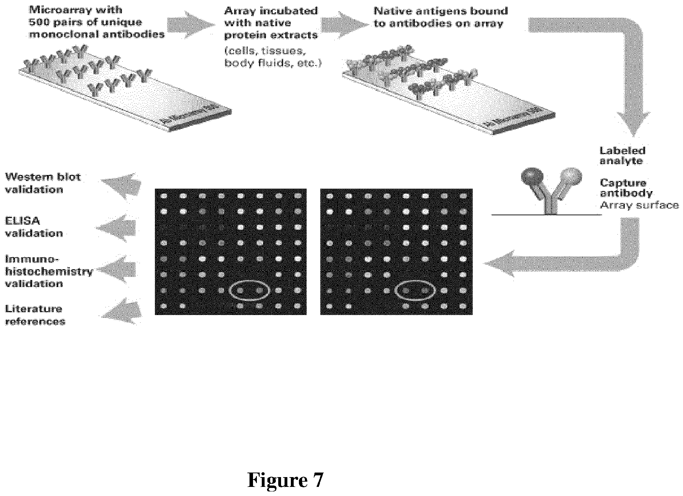

Reference is now made to **Figure 7** schematically illustrating an example of a preparation and analysis processes using protein microarrays consisting of antibodies bound to glass slides (chip). Such antibody chips are adjusted to profile changes in protein expression levels. According to a further embodiment, antibody microarrays are used to measure protein abundance in any biological sample, including cells, whole tissue and body fluids. The results may be picked up by a standard reader.

As shown in **Figure 7**, microarrays carrying predetermined unique monoclonal antibodies are prepared. The array is incubated with native protein extracts that may derive from cells, tissues, body fluids or any other biological sample. The native antigens are then allowed to be bound to their corresponding antibodies on the array. In the next step a labeled analyte, agent or entity may be added and the captured antibody complexes within the array surface are analysed by a fingerprinting technique. Fingerprinting techniques that can be used within the present invention include, but are not limited to, Western blot, ELAIZA, Immuno-histochemistry or any other fingerprinting technique known in the relevant literature.

It is also within the scope of the present invention that clinical trials are performed to determine the efficacy of the diagnostic kit (i.e. protein chip prototype) to predict the development of GVHD in human transplant patients.

In order to determine the fingerprint for GVHD, samples form humans are collected from sick and healthy people, and analyzed by mass spectrometry to identify the following:
- All the proteins that are present in the system
- Changes in protein expression
- All the RNA that is present in the system
- Changes in RNA expression
- Focusing on the DNA level

Human samples were collected using a CRA (clinical research associate) to recruit patients for a clinical trial (including Helsinki approval and negotiations with medical centers). The blood samples were collected from patients who have undergone bone marrow transplants.

In a preferred embodiment, a protein chip is utilized for analysis of the human samples to demonstrate its efficacy in predicting acute GVHD onset.

In a specific embodiment, the present invention provides a protein chip utilizing a large number of antibodies bound to a glass slide which can be used to determine the expression levels of different proteins in a given sample. One example is the development of a diagnostic kit based on a protein chip that can diagnose GVHD before the appearance of clinical symptoms

It is well within the scope of the present invention that the aforementioned kit contains a custom-made protein chip containing antibodies which are capable of monitoring the activation state and expression level of the GSK-3β, related pathways, apoptosis markers, proliferation markers, and others (taken from western blot, FACS, and fingerprinting data).

It is emphesised that the principal of the invention, namely GSK-3β and/or related proteins as diagnostic tool for abnormal cell proliferation, demonstrated the GVHD model, can be used to provide customized kit and method to detect and predict any other medical disorder associated with cell proliferation and/or the GSK-3β molecule. The kit analysis results are preferably compared against the diagnostic outcome (according to current/updated medical standards) for each patient.

In certain embodiments of the invention, the kit is calibrated by assaying human samples by western blot and/or other fingerprinting technique common in the relevant art.

### Example 3: Mass spectrometry experiment

### Goal:

To demonstrate Gsk-3β phosphorylation sites by PKC, thus allowing understanding the mechanisms of Gsk3β regulation used herein for diagnostic and therapeutic applications.

### Experimental procedure:

PKC and Gsk3β were incubated together in a test tube. For analysis of phosphorylated amino acids on Gsk3β, two samples were subjected to mass spectrometry:
- Experimental sample - PKC and Gsk3β incubated together in a kinase reaction buffer.
- Control sample - Gsk3β alone in a kinase reaction buffer, to determine if there are sites that are already phosphorylated or auto-phosphorylated.

### Results:

Nine phosphorylation sites were found on the Gsk3β incubated with PKC (experimental sample).

One of these sites (Thr324) was presented in the sample which contained Gsk3β alone (control sample), indicating that this site was previously phosphorylated or auto-phosphorylated.

The additional 8 sites on Gsk3β are shown by the current results to be phosphorylated directly by PKC.

Table 1 below summarizes the phosphrylation results of Gsk3β, showing 8 sites that are potentially phosphorylated by PKC *in vivo*. These phosphorylation sites are important in regulating Gsk3β.

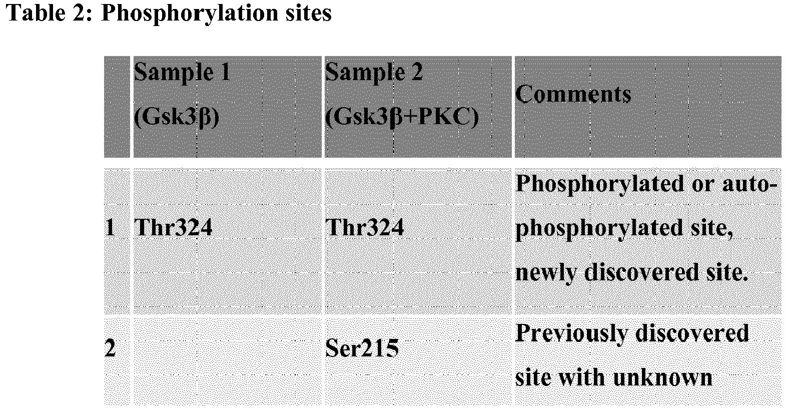

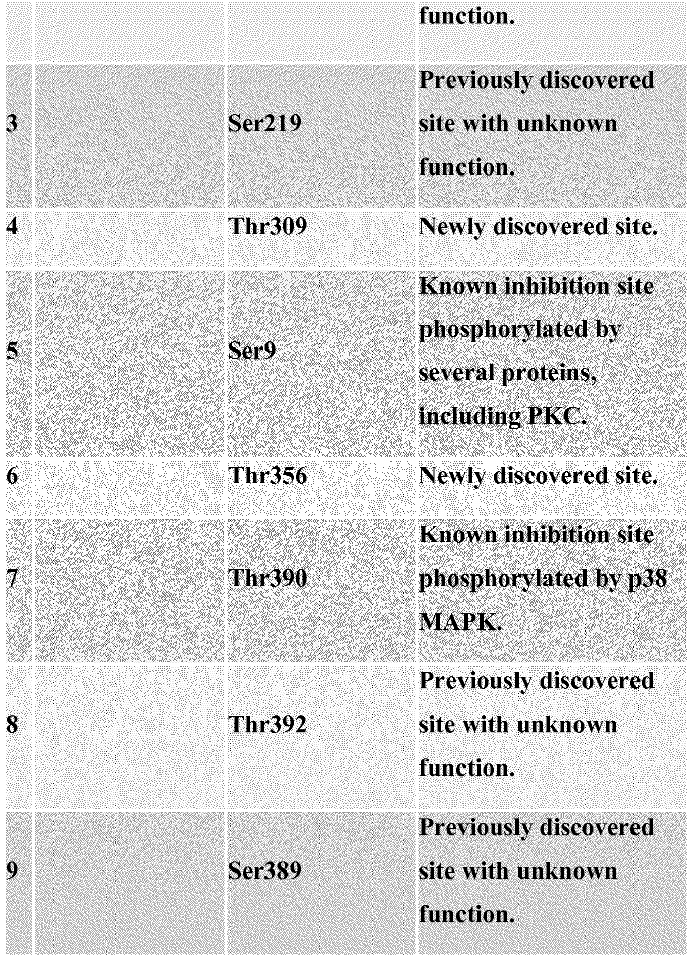

As shown in Table 1, three new phosphorylation sites were identified that have not been discovered previously, including a potential auto-phosphorylation site. More specifically, the 3 sites that were discovered as new phosphrylation sites of Gsk3β are Thr324, Thr309 and Thr356. In addition there are discovered sites which are with unknown function, namely, Ser215, Ser219, Thr392 and Ser389.

In a further embodiment of the invention, mass spectrometry is utilized to assay Gsk3β phosphorylation sites in human T-cells and thus assessing the effect of PKC on phosphorylation of the Gsk3β in human T cells.

### Example 4: Gsk3β affects T-cell proliferation

### Goal:

To determine the effect of PKC on Gsk3β and proliferation in human T-cells, in order to better understand the regulatory mechanisms of Gsk3β in T-cell proliferation. This data enables the development of tools as described *inter alia* to diagnose and treat medical disorders associated with cell proliferation and/or apoptosis such as GVHD.

### Experimental design:

In the following described experiment, these four major pathways were inhibited which directly phosphorylate Gsk3β in human T cells, preferably, Jurkat cells:
- P38 MAPK
- Erk
- PI-3K/Akt
- PKC

According to a certain embodiment, the following experimental groups were used:
1) Control group - untreated cells
2) Inhibitors group - treated with inhibitors against p38, Erk, and PI-3K pathways, with PKC unaffected.
3) PKC activated group - treated with inhibitors against p38, Erk, and PI-3K, and treated with PKC activator.
4) PKC inhibited group - treated with inhibitors against p38, Erk, and PI-3K, and treated with PKC inhibitor

### Results:

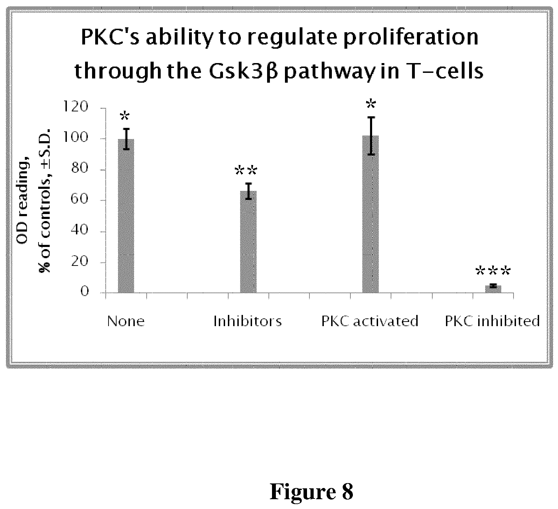

Reference is now made to **Figure 8** showing a graphic representation demonstrating the effect of PKC on regulation of T-cell proliferation through GSK-3β pathway. The results presented in this figure demonstrate that PKC is able to regulate proliferation through Gsk3β pathway in T-cells. The statistical differences are calculated by One-way ANOVA (Student-Newman-Keuls Method), when the p-value<0.01. Within the graphic representation, the statistical differences are indicated by number of stars.

As shown in Figure 7, the diagram is divided into 4 main columns:
**None:** Untreated control group, the values of which is set as 100%.
**Inhibitors group:** Inhibition of 3 different pathways, namely p38, Erk, and PI-3K pathways, significantly reduced cell proliferation in comparison to the control group. These results indicate that lower levels of Gsk3β phosphorylation is correlated with decreased cell proliferation.
**PKC group:** Activation of PKC, which is shown by the present invention to phosphorylate Gsk3β (Example 2) restores the cells to a "normal" proliferation level, which is similar to the control, despite the presence of other inhibitors. These results indicate that Gsk3β phosphorylation is correlated with an increase in proliferation.
**PKC inhibited group:** When all of the 4 major pathways are inhibited (and thus Gsk3β is not phosphorylated), there is almost no cell proliferation. These results indicate that Gsk3β regulates T-cell proliferation.

### Conclusions:

Of the above described experiments it can be concluded that:
- Gsk3β sites which are phosphorylated by PKC may play a role in Gsk3β regulation.
- These sites can be potentially used to monitor the activation state of Gsk3β, which is herein used for diagnostic and therapeutic purposes.
- PKC works through Gsk3β to regulate T-cell proliferation.
- PKC inhibition and inhibition of other pathways which regulate Gsk3β, are shown to almost completely abrogate T-cell proliferation.
- PKC can potentially be used to more accurately predict Gsk3β activity levels, T-cell proliferation, and to predict pathological conditions and diseases associated with T-cell proliferation, such as the prediction of GVHD onset.
- The affect of Gsk3β and PKC on T-cell proliferation in living cells, is further assessed by assaying (i.e. by Western blotting):
   ∘ total Gsk3β protein;
   ∘ Gsk3β ser9 phosphorylation- indicating Gsk3β inactivation;
   ∘ PKC activation- indicating of Gsk3β inactivation;
   ∘ b-catenin - a measure of the inactivation of the Gsk3β through the activated Wnt pathway;
   ∘ Dvl - a protein upstream of Gsk3β in the Wnt pathway, and an indicator of Wnt pathway activation;
   ∘ Erk - Map kinase signaling cascade protein that directly phosphorylates Gsk3β at Ser9;
   ∘ P38 MAPK - Map kinase signaling cascade protein that directly phosphorylates Gsk3β at Ser389;
   ∘ Gsk3β tyr216 Phosphorylation - Indicates activation of Gsk3β; and,
   ∘ House keeping protein - loading control

The above described studies provide a better understanding of the role of Gsk3β in T-cell proliferation and subsequently in early diagnosis and prevention of diseases associated with T-cell proliferation such as GVHD development.

In a further aspect of the invention, the affect of the newly identified phosphorylation sites on the Gsk3β on the activation state of the Gsk3β, is determined.

In a specific embodiment, Gsk3β activity is determined by assaying the levels of Gsk3β substrates.

### Example 5: Diagnostic kit

A description of a GVHD diagnostic kit is provided as an example of a diagnostic kit prepared by the method and guidance of the present invention that can be tailored to diagnose any pathological disorder associated with cell proliferation or apoptosis that is herein shown for the first time to be linked with Gsk3β and related proteins.
Name -GVHD early diagnostic kit, version 1.0
Contains- Protein chip, i.e. glass slide with attached antibodies
Application- Early diagnosis of GVHD before appearance of symptoms
Usage- Samples collected by blood tests
Procedures- Each kit contains materials for 7 tests, to be done over time
Target Population- Hematopoietic Stem Cell (including bone marrow transplants) Transplant recipients.

The advantages of such a GVHD diagnostic kit:
Early Diagnosis: The GVHD diagnostic kit allows for the diagnosis of GVHD prior to appearance of observable symptoms. Thus the GVHD diagnostic kit saves lives.
   - Minimally Invasive: The diagnostic kit is based on a series of simple blood tests, as opposed to the current method of biopsy of affected tissues.
   - Real-time Information: The GVHD diagnostic kit gives the doctor information in real-time about the patient's condition.
   - Better Treatment Options: This kit enables doctors to treat patients earlier, when treatments are more effective and can potentially prevent GVHD development altogether.
   - Reduced Costs: The diagnostic kit allows reduce costs to doctors, hospitals, and patients, by reducing hospital stays, expensive tests, and expensive treatments.

A kit based on the platform technology as provided by the present invention can be applied for the diagnosis and/ or prevention of pathological disorders associated with T-cell proliferation, for example: autoimmune disorders, inflammatory disease, tissue rejection, cancer, etc.

Diagnostic kits based on the technology of the present invention can be used in companion diagnosis approaches, by preferably performing the following steps: (1) Monitoring changes in activation state of Gsk3β and related proteins allows diagnosing other disorders. (2) Unique protein chips can be developed based on the platform technology of the present invention, which are custom-designed for each disorder. (3) Additional software packages are developed for analysis of each individual disease. (4) Disease treatments are screened using protein chips to ensure the drug is effectively targeting the molecular causes of the disease. (5) The understanding of the molecular pathways involved in each disease allows developing new specialized drugs.

### References

1. Miura T, Miki T. GSK-3beta, a therapeutic target for cardiomyocyte protection. Circ J. 2009
2. Sugden PH, Fuller SJ, Weiss SC, Clerk A. Glycogen synthase kinase 3 (GSK3β) in the heart: a point of integration in hypertrophic signaling and a therapeutic target? A critical analysis. Br J Pharmacol 153: S137-S153. 2008
3. Jin J, Wang GL, Salisbury E, Timchenko L, Timchenko NA. GSK3beta-cyclin D3-CUGBP1-eIF2 pathway in aging and in myotonic dystrophy. Cell Cycle, 2009
4. Kerkela R, Woulfe K, Force T. Glycogen synthase kinase-3b - actively inhibiting hypertrophy. Trends Cardiovasc Med 17: 91-96. 2007
5. Ross SE, Erickson RL, Hemati N, MacDougald OA. Glycogen synthase kinase 3 is an insulin-regulated C/EBPa kinase. Mol Cell Biol 1999
6. Hoeppner LH, Secreto FJ, Westendorf JJ. Wnt signaling as a therapeutic target for bone diseases. Expert Opin Ther Targets. 2009
7. Juhaszova M, Zorov DB, Yaniv Y, Nuss HB, Wang S, Sollott SJ. Role of glycogen synthase kinase-3beta in cardioprotection. Circ Res. 2009
8. Tsai S-J, et al. Glycogen synthase kinase-3beta gene is associated with antidepressant treatment response in Chinese major depressive disorder. Pharmacogenomics J. 2008
9. Zhang K, Yang C, Xu Y, Sun N, Yang H, Liu J, Xu Q, Shen Y.J Neural Transm. Genetic association of the interaction between the BDNF and GSK3B genes and major depressive disorder in a Chinese population. J Neural Transm. 117(3):393-401 (2010).
10. Inkster B, Nichols TE, Saemann PG, Auer DP, Holsboer F, Muglia P, Matthews PM.Arch Association of GSK3beta polymorphisms with brain structural changes in major depressive disorder. Gen Psychiatry. 66(7):721-8 (2009).
11. Benedetti, F. et al. A glycogen synthase kinase 3-beta promoter gene single nucleotide polymorphism is associated with age at onset and response to total sleep deprivation in bipolar depression. Neuroscience letters. 368(2): 123-6 (2004).
12. Benedetti, F. et al. A single nucleotide polymorphism in glycogen synthase kinase 3-beta promoter gene influences onset of illness in patients affected by bipolar disorder. Neuroscience letters. Jan;355(2-Jan):37-40 (2004).
13. Lachman HM, Pedrosa E, Petruolo OA, Cockerham M, Papolos A, Novak T, Papolos DF, Stopkova P. Increase in GSK3beta gene copy number variation in bipolar disorder. Am J Med Genet B Neuropsychiatr Genet. 144B(3):259-65 (2007).
14. Smith E, Frenkel B. Glucocorticoids inhibit the transcriptional activity of LEF/TCF in differentiating osteoblasts in a glycogen synthase kinase-3beta-dependent and -independent manner. J Biol Chem 280: 2388-2394. 2005
15. Dokken BB, Sloniger JA, Henriksen EJ. Acute selective glycogen synthase kinase-3 inhibition enhances insulin signaling in prediabetic insulin-resistant rat skeletal muscle. Am J Physiol Endocrinol Metab 288: E1188-E1194. (2005)
16. Andrade Filho PA, Letra A, Cramer A, Prasad JL, Garlet GP, Vieira AR, Ferris RL, Menezes R. Insights from studies with oral cleft genes suggest associations between WNT-pathway genes and risk of oral cancer. J Dent Res. 90(6):740-6. 2011
17. Robertson LA, Kim AJ, Werstuck GH. Mechanisms linking diabetes mellitus to the development of atherosclerosis: a role for endoplasmic reticulum stress and glycogen synthase kinase-3. Can J Physiol Pharmacol 84: 39-48. 2006
18. Ring DB, Johnson KW, Henriksen EJ, Nuss JM, Goff D, Kinnick TR et al. Selective glycogen synthase kinase 3 inhibitors potentiate insulin activation of glucose transport and utilization in vitro and in vivo. Diabetes 52: 588-595. 2003
19. Miura T, Tanno M. Mitochondria and GSK-3beta in cardioprotection against ischemia/reperfusion injury. Cardiovasc Drugs Ther. 2010
20. Ougolkov AV, Fernandez-Zapico ME, Bilim VN, Smyrk TC, Chari ST, Billadeau DD.Clin Aberrant nuclear accumulation of glycogen synthase kinase-3beta in human pancreatic cancer: association with kinase activity and tumor dedifferentiation. Cancer Res. 12(17):5074-81, 2006.
21. Ban KC, Singh H, Krishnan R, Seow HF. GSK-3beta phosphorylation and alteration of beta-catenin in hepatocellular carcinoma. Cancer Lett. Sep 25;199(2):201-8. 2003.
22. Nikoulina SE, Ciaraldi TP, Mudailar S, Mohideen P, Carter L, Henry RR. Potential role of GSK-3 in skeletal muscle insulin resistance Type 2 diabetes. Diabetes 49:263-271, 2000.
23. Hardt SE, Sadoshima Glycogen synthase kinase-3beta: a novel regulator of cardiac hypertrophy and development. J.Circ Res. 2002 May 31;90(10):1055-63. 2002
24. Wang Zhong, et al.Glycogen synthase kinase 3 in MLL leukaemia maintenance and targeted therapy. Nature. 2008
25. Leis H, Segrelles C, Ruiz S, Santos M, Paramio JM: Expression, localization, and activity of glycogen synthase kinase 3beta during mouse skin tumorigenesis. Mol Carcinog 2002, 35:180-185. 2002
26. Ma C, Wang J, Gao Y, Gao TW, Chen G, Bower KA, Odetallah M, Ding M, Ke Z, Luo J: The role of glycogen synthase kinase 3beta in the transformation of epidermal cells. Cancer Res 2007
27. Kang T, Wei Y, Honaker Y, Yamaguchi H, Appella E, Hung MC, Piwnica-Worms H: GSK-3 beta targets Cdc25A for ubiquitin-mediated proteolysis, and GSK-3 beta inactivation correlates with Cdc25A overproduction in human cancers. Cancer Cell 2008
28. Bauer K, Dowejko A, Bosserhoff AK, Reichert TE, Bauer RJ: P-cadherin induces an epithelial-like phenotype in oral squamous cell carcinoma by GSK-3beta-mediated Snail phosphorylation. Carcinogenesis 2009
29. Wang Y, Lam JB, Lam KS, Liu J, Lam MC, Hoo RL, Wu D, Cooper GJ, Xu A: Adiponectin modulates the glycogen synthase kinase-3beta/beta-catenin signaling pathway and attenuates mammary tumorigenesis of MDA-MB-231 cells in nude mice. Cancer Res 2006
30. Dong J, Peng J, Zhang H, Mondesire WH, Jian W, Mills GB, Hung MC, Meric-Bernstam F: Role of glycogen synthase kinase 3beta in rapamycin-mediated cell cycle regulation and chemosensitivity. Cancer Res 2005
31. Farago M, Dominguez I, Landesman-Bollag E, Xu X, Rosner A, Cardiff RD, Seldin DC: Kinase-inactive glycogen synthase kinase 3beta promotes Wnt signaling and mammary tumorigenesis. Cancer Res 2005
32. Dal Col J, Dolcetti R: GSK-3beta inhibition: at the crossroad between Akt and mTOR constitutive activation to enhance cyclin D1 protein stability in mantle cell lymphoma. Cell Cycle 2008
33. Ding Q, He X, Xia W, Hsu JM, Chen CT, Li LY, Lee DF, Yang JY, Xie X, Liu JC, Hung MC: Myeloid cell leukemia-1 inversely correlates with glycogen synthase kinase-3beta activity and associates with poor prognosis in human breast cancer. Cancer Res 2007
34. Mai W, Miyashita K, Shakoori A, Zhang B, Yu ZW, Takahashi Y, Motoo Y, Kawakami K, Minamoto T: Detection of active fraction of glycogen synthase kinase 3beta in cancer cells by nonradioisotopic in vitro kinase assay. Oncology 2006
35. Garcea G, Manson MM, Neal CP, Pattenden CJ, Sutton CD, Dennison AR, Berry DP: Glycogen synthase kinase-3 beta; a new target in pancreatic cancer? Curr Cancer Drug Targets 2007
36. Zheng H, Saito H, Masuda S, Yang X, Takano Y: Phosphorylated GSK3beta-ser9 and EGFR are good prognostic factors for lung carcinomas. Anticancer Res 2007
37. Tian D, Zhu M, Chen WS, Li JS, Wu RL, Wang X: Role of glycogen synthase kinase 3 in squamous differentiation induced by cigarette smoke in porcine tracheobronchial epithelial cells.Food Chem Toxicol 2006
38. Shakoori A, Mai W, Miyashita K, Yasumoto K, Takahashi Y, Ooi A, Kawakami K, Minamoto T: Inhibition of GSK-3 beta activity attenuates proliferation of human colon cancer cells in rodents. Cancer Sci 2007
39. Mamaghani S, Patel S, Hedley DW: Glycogen synthase kinase-3 inhibition disrupts nuclear factor-kappaB activity in pancreatic cancer, but fails to sensitize to gemcitabine chemotherapy. BMC Cancer 2009
40. Morrison JA, Gulley ML, Pathmanathan R, Raab-Traub N: Differential signaling pathways are activated in the Epstein-Barr virus-associated malignancies nasopharyngeal carcinoma and Hodgkin lymphoma. Cancer Res 2004
41. Ghosh JC, Altieri DC: Activation of p53-dependent apoptosis by acute ablation of glycogen synthase kinase-3beta in colorectal cancer cells. Clin Cancer Res 2005
42. Abrahamsson AE, Geron I, Gotlib J, Dao KH, Barroga CF, Newton IG, Giles FJ, Durocher J, Creusot RS, Karimi M, et al.: Glycogen synthase kinase 3beta missplicing contributes to leukemia stem cell generation. Proc Natl Acad Sci USA 2009,
43. Zhou Y, Uddin S, Zimmerman T, Kang JA, Ulaszek J, Wickrema A: Growth control of multiple myeloma cells through inhibition of glycogen synthase kinase-3. Leuk Lymphoma 2008
44. Ougolkov AV, Fernandez-Zapico ME, Savoy DN, Urrutia RA, Billadeau DD: Glycogen synthase kinase-3beta participates in nuclear factor kappaB-mediated gene transcription and cell survival in pancreatic cancer cells. Cancer Res 2005
45. Cao Q, Lu X, Feng YJ: Glycogen synthase kinase-3beta positively regulates the proliferation of human ovarian cancer cells. Cell Res 2006
46. Ougolkov AV, Bone ND, Fernandez-Zapico ME, Kay NE, Billadeau DD: Inhibition of glycogen synthase kinase-3 activity leads to epigenetic silencing of nuclear factor kappaB target genes and induction of apoptosis in chronic lymphocytic leukemia B cells. Blood 2007
47. Adler JT, Cook M, Luo Y, Pitt SC, Ju J, Li W, Shen B, Kunnimalaiyaan M, Chen H: Tautomycetin and tautomycin suppress the growth of medullary thyroid cancer cells via inhibition of glycogen synthase kinase-3beta. Mol Cancer Ther 2009f
48. Kotliarova S, Pastorino S, Kovell LC, Kotliarov Y, Song H, Zhang W, Bailey R, Maric D, Zenklusen JC, Lee J, Fine HA: Glycogen synthase kinase-3 inhibition induces glioma cell death through c-MYC, nuclear factor-kappaB, and glucose regulation. Cancer Res 2008
49. Mai W, Kawakami K, Shakoori A, Kyo S, Miyashita K, Yokoi K, Jin M, Shimasaki T, Motoo Y, Minamoto T: Deregulated GSK3 {beta} sustains gastrointestinal cancer cells survival by modulating human telomerase reverse transcriptase and telomerase. Clin Cancer Res 2009
50. Sun A, Shanmugam I, Song J, Terranova PF, Thrasher JB, Li B: Lithium suppresses cell proliferation by interrupting E2F-DNA interaction and subsequently reducing S-phase gene expression in prostate cancer. Prostate 2007
51. Bilim V, Ougolkov A, Yuuki K, Naito S, Kawazoe H, Muto A, Oya M, Billadeau D, Motoyama T, Tomita Y: Glycogen synthase kinase-3: a new therapeutic target in renal cell carcinoma Br J Cancer 2009
52. Goodarzi MO, Antoine HJ, Pall M, Cui J, Guo X, Azziz R. Preliminary evidence of glycogen synthase kinase 3 beta as a genetic determinant of polycystic ovary syndrome. Fertil Steril. 87(6):1473-6, 2007
53. Hosgood HD 3rd, Menashe I, Shen M, Yeager M, Yuenger J, Rajaraman P, He X, Chatterjee N, Caporaso NE, Zhu Y, Chanock SJ, Zheng T, Lan Q. Pathway-based evaluation of 380 candidate genes and lung cancer susceptibility suggests the importance of the cell cycle pathway. Carcinogenesis.29(10):1938-43, 2008
54. Durie BG, Van Ness B, Ramos C, Stephens O, Haznadar M, Hoering A, Haessler J, Katz MS, Mundy GR, Kyle RA, Morgan GJ, Crowley J, Barlogie B, Shaughnessy J Jr. Genetic polymorphisms of EPHX1, Gsk3beta, TNFSF8 and myeloma cell DKK-1 expression linked to bone disease in myeloma. Leukemia. 23(10):1913-9. 2009.
55. Galimberti D, Macmurray J, Scalabrini D, Fenoglio C, De Riz M, Comi C, Comings D, Cortini F, Villa C, Serpente M, Cantoni C, Ridolfi E, Fardipoor MH, Leone M, Monaco F, Bresolin N, Scarpini E.GSK3β genetic variability in patients with Multiple Sclerosis. Neurosci Lett. 497(1):46-8. 2011.
56. De Ferrari, G. V. & Inestrosa, N. C. Wnt signaling function in Alzheimer's disease. Brain Res. Rev. 33, 1-12 (2000).
57. Mateo I, Infante J, Llorca J, Rodriguez E, Berciano J, Combarros O. Association between Glycogen Synthase Kinase-3beta Genetic Polymorphism and Late-Onset Alzheimer's Disease. Dement Geriatr Cogn Disord 21(4) 228-232 (2006)
58. Kwok JB, Loy CT, Hamilton G, Lau E, Hallupp M, Williams J, Owen MJ, Broe GA, Tang N, Lam L, Powell JF, Lovestone S, Schofield PR. Glycogen synthase kinase-3beta and tau genes interact in Alzheimer's disease. Ann Neurol. 64(4):446-54, 2008.
59. Zhang N, Yu JT, Yang Y, Yang J, Zhang W, Tan L.. Association analysis of GSK3B and MAPT polymorphisms with Alzheimer's disease in Han Chinese. Brain Res. 1391:147-53 (2011).
60. Hanger DP, Hughes K, Woodgett JR, Brion JP, Anderton BH. Glycogen synthase kinase-3 induces Alzheimer's disease-like phosphorylation of tau: generation of paired helical filament epitopes and neuronal localisation of the kinase. Neurosci Lett 147: 58-62. 1992
61. Takashima A. GSK-3 is essential in the pathogenesis of Alzheimer's disease. J Alzheimers Dis 9: 309-317. 2006
62. Hernández F, Avila J. The role of glycogen synthase kinase 3 in the early stages of Alzheimers' disease. FEBS Lett 582: 3848-3854. 2008
63. Jope RS, Roh MS. Glycogen synthase kinase-3 (GSK3β) in psychiatric diseases and therapeutic interventions. Curr Drug Targets 7: 1421-1434. 2006
64. Schaffer BA, Bertram L, Miller BL, Mullin K, Weintraub S, Johnson N, Bigio EH, Mesulam M, Wiedau-Pazos M, Jackson GR, Cummings JL, Cantor RM, Levey AI, Tanzi RE, Geschwind DH. Association of GSK3B with Alzheimer disease and frontotemporal dementia. Arch Neurol. 65(10):1368-74 (2008).
65. Kwok, J.B., Hallupp, M., Loy, C.T., Chan, D.K., Woo, J., Mellick, G.D., Buchanan, D.D., Silburn, P.A., Halliday, G.M., Schofield, P.R. GSK3B polymorphisms alter transcription and splicing in Parkinson's disease. Ann. Neurol. 2005
66. Das G, Misra AK, Das SK, Ray K, Ray J. Role of tau kinases (CDK5R1 and GSK3B) in Parkinson's disease: A study from India. Neurobiol Aging. 2010
67. Dugo L, Collin M, Thiemermann C. Glycogen synthase kinase 3beta as a target for the therapy of shock and inflammation. Shock. 2007
68. Pizarro JG, Folch J, Esparza JL, Jordan J, Pallas M, Camins A. A molecular study of pathways involved in the inhibition of cell proliferation in neuroblastoma B65 cells by the GSK-3 inhibitors lithium and SB-415286. J Cell Mol Med. 2009
69. Valencia A, Reeves PB, Sapp E, Li X, Alexander J, Kegel KB, Chase K, Aronin N, DiFiglia M. Mutant huntingtin and glycogen synthase kinase 3-beta accumulate in neuronal lipid rafts of a presymptomatic knock-in mouse model of Huntington's disease. J Neurosci Res. 2010

### References

**1** Hanger, D. P., Hughes, K., Woodgett, J. R., Brion, J. P. and Anderton, B. H. (1992). Glycogen synthase kinase-3 induces Alzheimer's disease-like phosphorylation of tau: generation of paired helical filament epitopes and neuronal localisation of the kinase. Neurosci Lett. 147,58 -62.
**2** de Groot, R. P., Auwerx, J., Bourouis, M., and Sassone-Corsi, P. (1992) Negative regulation of Jun/AP-1: conserved function of glycogen synthase kinase 3 and the Drosophila kinase shaggy. Oncogene 7, 841-847.
**3** Welsh, G. I., and Proud, C. G. (1993) Glycogen synthase kinase-3 is rapidly inactivated in response to insulin and phosphorylates eukaryotic initiation factor elF-2B. Biochem. J. 294, 625-629.
**4** Sears, R. et al. (2000) Multiple Ras-dependent phosphorylation pathways regulate Myc protein stability. Genes Dev. 14, 2501-2514.
**5** Gross I, et al. (2005) Phosphorylation of the homeotic tumor suppressor Cdx2 mediates its ubiquitin-dependent proteasome degradation. Oncogene 24, 7955-63
**6** Fiol CJ, et al. Formation of protein kinase recognition sites by covalent modification of the substrate. Molecular mechanism for the synergistic action of casein kinase II and glycogen synthase kinase 3. J. Biol. Chem., 262 (1987), pp. 14042-14048.
**7** Hughes, K., Ramakrishna, S., Benjamin, W. B. and Woodgett, J. R. (1992). Identification of multifunctional ATP-citrate lyase kinase as the alpha-isoform of glycogen synthase kinase-3. Biochem. J. 288,309 -314.
**8** Ikeda S., et al. Axin, a negative regulator of the Wnt signaling pathway, forms a complex with GSK-3β and β-catenin and promotes GSK-3β-dependent phosphorylation of β-catenin. EMBO J., 17 (1998), pp. 1371-1384.
**9** Quélo I, Akhouayri O, Prud'homme J, St-Arnaud R (2004) GSK3 beta-dependent phosphorylation of the alpha NAC coactivator regulates its nuclear translocation and proteasome-mediated degradation. Biochemistry 43, 2906-14
**10** Fiol C. J., Williams, J. S., Chou, C. H., Wang, Q. M., Roach, P. J. and Andrisani, O. M. (1994). A secondary phosphorylation of CREB341 at Ser129 is required for the cAMP-mediated control of gene expression. A role for glycogen synthase kinase-3 in the control of gene expression. J. Biol. Chem. 269,32187 -32193.
**11**Twomey C and McCarthy VJ. (2006). Presenilin-1 is an unprimed glycogen synthase kinase-3beta substrate. FEBS Lett., 580, pp. 4015-4020
**12** Rubinfeld, B., Albert, I., Porfiri, E., Fiol, C., Munemitsu, S. and Polakis, P. (1996). Binding of GSK3beta to the APC-beta-catenin complex and regulation of complex assembly. Science 272,1023 -1026.
**13** Chen MC, et al. (2011) Novel mechanism by which histone deacetylase inhibitors facilitate topoisomerase IIα degradation in hepatocellular carcinoma cells. Hepatology 53, 148-59
**14** Mao Y, et al. (2009) Disrupted in schizophrenia 1 regulates neuronal progenitor proliferation via modulation of GSK3beta/beta-catenin signaling. Cell 136, 1017-31
**15** Chu, B., Soncin, F., Price, B. D., Stevenson, M. A. and Calderwood, S. K. (1996). Sequential phosphorylation by mitogen-activated protein kinase and glycogen synthase kinase 3 represses transcriptional activation by heat shock factor-1. J. Biol. Chem. 271,30847 -30857.
**16** Y Hong, et al. (2000). Cloning and characterization of a novel human ninein protein that interacts with the glycogen synthase kinase 3β. Biochim. Biophys. Acta, 1492 , pp. 513-516.
**17** Beals, C. R., Sheridan, C. M., Turck, C. W., Gardner, P. and Crabtree, G. R. (1997). Nuclear export of NF-ATc enhanced by glycogen synthase kinase-3. Science 275,1930 -1934.
**18** Li, Y., Bharti, A., Chen, D., Gong, J. and Kufe, D. (1998). Interaction of glycogen synthase kinase 3beta with the DF3/MUC1 carcinoma-associated antigen and beta-catenin. Mol. Cell. Biol. 18,7216 -7224.
**19** Eldar-Finkelman, H. and Krebs, E. G. (1997). Phosphorylation of insulin receptor substrate 1 by glycogen synthase kinase 3 impairs insulin action. Proc. Natl. Acad. Sci. USA 94,9660 - 9664.
**20** Ross, S. E., Erickson, R. L., Hemati, N. and MacDougald, O. A. (1999) Glycogen synthase kinase 3 is an insulin-regulated C/EBPalpha kinase. Mol. Cell. Biol. 19, 8433-8441
**21** Hemmings BA, et al. Phosphorylation of the type-II regulatory subunit of cyclic-AMP-dependent protein kinase by glycogen synthase kinase 3 and glycogen synthase kinase 5. Eur. J. Biochem., 127 (1982), pp. 473-481.
**22** Al-Khouri AM, et al. (2005) Cooperative phosphorylation of the tumor suppressor phosphatase and tensin homologue (PTEN) by casein kinases and glycogen synthase kinase 3beta. J Biol Chem 280, 35195-202
**23** C.J Fiol, J.H Haseman, Y.H Wang, P.J Roach, R.W Roeske, M Kowalczuk and A.A DePaoli-Roach, Phosphoserine as a recognition determinant for glycogen synthase kinase-3: phosphorylation of a synthetic peptide based on the G-component of protein phosphatase-1. Arch. Biochem. Biophys., 267 (1988), pp. 797-802.
**24** Neumann M, et al. (2011) Glycogen synthase kinase-3β is a crucial mediator of signal-induced RelB degradation. Oncogene 30, 2485-92
**25** CSanchez, J Diaz-Nido and J Avila, Phosphorylation of microtubule-associated protein 2 (MAP2) and its relevance for the regulation of the neuronal cytoskeleton function. Prog. Neurobiol., 61 (2000), pp. 133-168.
**26** Yang SD. (1986). Identification of the ATP.Mg-dependent protein phosphatase activator (FA) as a myelin basic protein kinase in the brain. J. Biol. Chem., 261 , pp. 11786-11791.
**27** M Hoshi, A Takashima, K Noguchi, M Murayama, M Sato, S Kondo, Y Saitoh, K Ishiguro, T Hoshino and K Imahori, Regulation of mitochondrial pyruvate dehydrogenase activity by tau protein kinase l/glycogen synthase kinase 3β in brain. Proc. Natl. Acad. Sci. U.S.A., 93 (1996), pp. 2719-2723.
**28** Rada P, et al. (2011) SCF/{beta}-TrCP Promotes Glycogen Synthase Kinase 3-Dependent Degradation of the Nrf2 Transcription Factor in a Keap1-Independent Manner. Mol Cell Biol 31, 1121-33
**29** K Mackie, B.C Sorkin, A.C Nairn, P Greengard, G.M Edelman and B.A Cunningham, Identification of two protein kinases that phosphorylate the neural cell-adhesion molecule, N-CAM. J. Neurosci., 9 (1989), pp. 1883-1896.
**30** Galliher-Beckley AJ, Williams JG, Collins JB, Cidlowski JA (2008) Glycogen Synthase Kinase 3{beta}-Mediated Serine Phosphorylation of the Human Glucocorticoid Receptor Redirects Gene Expression Profiles. Mol Cell Biol 28, 7309-22
**31** Sasaki T, Ishiguro K, Hisanaga S (2009) Novel axonal distribution of neurofilament-H phosphorylated at the glycogen synthase kinase 3beta-phosphorylation site in its E-segment. J Neurosci Res 87, 3088-97
**32** Khaled, M., Larribere, L., Bille, K., Aberdam, E., Ortonne, J. P., Ballotti, R. and Bertolotto, C. (2002). Glycogen synthase kinase 3b is activated by cAMP and plays an active role in the regulation of melanogenesis. J. Biol. Chem. 277,33690 -33697.
**33** Morfini, G., Szebenyi, G., Elluru, R., Ratner, N. and Brady, S. T. (2002). Glycogen synthase kinase 3 phosphorylates kinesin light chains and negatively regulates kinesin-based motility. EMBO J. 21,281 -293.
**34** J.C Bournat, A.M.C Brown and A.P Soler, Wnt-1 dependent activation of the survival factor NF-κB in PC12 cells. J. Neurosci. Res., 61 (2000), pp. 21-32.
**35** Takeshi Yoshimura, Yoji Kawano, Nariko Arimura, Saeko Kawabata, Akira Kikuchi, Kozo Kaibuchi (2005). GSK-3β Regulates Phosphorylation of CRMP-2 and Neuronal Polarity. Cell Vol. 120, Issue 1, pp. 137-149
**36** Riobó NA, Lu K, Ai X, Haines GM, Emerson CP Jr. Phosphoinositide 3-kinase and Akt are essential for Sonic Hedgehog signaling. Proc Natl Acad Sci U S A. 2006 Mar 21;103(12):4505-10.
**37** Zou C, et al. (2011) LPS Impairs Phospholipid Synthesis by Triggering {beta}-Transducin Repeat-containing Protein ({beta}-TrCP)-mediated Polyubiquitination and Degradation of the Surfactant Enzyme Acyl-CoA:Lysophosphatidylcholine Acyltransferase I (LPCAT1). J Biol Chem 286, 2719-27
**38** Kulikov R, Boehme KA, Blattner C. Glycogen synthase kinase 3-dependent phosphorylation of Mdm2 regulates p53 abundance. Mol Cell Biol. 2005 Aug;25(16):7170-80.
**39** Lee JY, Yu SJ, Park YG, Kim J, Sohn J. Glycogen synthase kinase 3beta phosphorylates p21WAF1/CIP1 for proteasomal degradation after UV irradiation. Mol Cell Biol. 2007 Apr;27(8):3187-98
**40** Diehl, J. A., Cheng, M., Roussel, M. F. & Sherr, C. J. (1998) Glycogen synthase kinase-3beta regulates cyclin D1 proteolysis and subcellular localization. Genes Dev. 12, 3499-3511.
**41** Qu L, et al. (2004) Endoplasmic reticulum stress induces p53 cytoplasmic localization and prevents p53-dependent apoptosis by a pathway involving glycogen synthase kinase-3beta. Genes Dev 18, 261-77
**42** Welcker M, et al. (2003) Multisite phosphorylation by Cdk2 and GSK3 controls cyclin E degradation. Mol Cell 12, 381-92
**43** Y.R Hong, C.H Chen, D.S Cheng, S.L Howng and C.C Chow, (1998). Human dynamin-like protein interacts with the glycogen synthase kinase 3β. Biochem. Biophys. Res. Commun., 249 pp. 697-703.
**44** Krymsky MA, Kudryashov DS, Shirinsky VP, Lukas TJ, Watterson DM, Vorotnikov AV. Phosphorylation of kinase-related protein (telokin) in tonic and phasic smooth muscles. J Muscle Res Cell Motil. 2001;22(5):425-37.
**45** Michalak M, Fu SY, Milner RE, Busaan JL, Hance JE. Phosphorylation of the carboxyl-terminal region of dystrophin. Biochem Cell Biol. 1996;74(4):431-7.
**46** Chen L, Salinas GD, Li X (2009) Regulation of serotonin 1 B receptor by glycogen synthase kinase-3. Mol Pharmacol 76, 1150-61
**47** Fei E, et al. (2007) Phosphorylation of ataxin-3 by glycogen synthase kinase 3beta at serine 256 regulates the aggregation of ataxin-3. Biochem Biophys Res Commun 357, 487-92
**48** Linseman DA, et al. (2004) Glycogen synthase kinase-3beta phosphorylates Bax and promotes its mitochondrial localization during neuronal apoptosis. J Neurosci 24, 9993-10002
**49** Sapkota G, et al. (2007) Balancing BMP signaling through integrated inputs into the Smad1 linker. Mol Cell 25, 441-54
**50** Sahar S, et al. (2010) Regulation of BMAL1 protein stability and circadian function by GSK3beta-mediated phosphorylation. PLoS One 5, e8561
**51** Hsu HC, et al. (2005) Characterization of two non-testis-specific CABYR variants that bind to GSK3beta with a proline-rich extensin-like domain. Biochem Biophys Res Commun 329, 1108-17
**52** Zeng X, et al. (2005) A dual-kinase mechanism for Wnt co-receptor phosphorylation and activation. Nature 438, 873-7
**53** Inoki K, et al. (2006) TSC2 integrates Wnt and energy signals via a coordinated phosphorylation by AMPK and GSK3 to regulate cell growth. Cell 126, 955-68
**54** Lickert H, Bauer A, Kemler R, Stappert J (2000) Casein kinase II phosphorylation of E-cadherin increases E-cadherin/beta-catenin interaction and strengthens cell-cell adhesion. J Biol Chem 275, 5090-5
**55** Kitagawa K, et al. (2009) Fbw7 promotes ubiquitin-dependent degradation of c-Myb: involvement of GSK3-mediated phosphorylation of Thr-572 in mouse c-Myb. Oncogene 28, 2393-405
**56** Xu Y, Harton JA, Smith BD (2008) CIITA mediates interferon-gamma repression of collagen transcription through phosphorylation-dependent interactions with co-repressor molecules. J Biol Chem 283, 1243-56
**57** Spengler ML, Kuropatwinski KK, Schumer M, Antoch MP (2009) A serine cluster mediates BMAL1-dependent CLOCK phosphorylation and degradation. Cell Cycle 8, 4138-46
**58** Harada Y, et al. (2005) Ser-557-phosphorylated mCRY2 is degraded upon synergistic phosphorylation by glycogen synthase kinase-3 beta. J Biol Chem 280, 31714-21
**59** Xia X, Mariner DJ, Reynolds AB (2003) Adhesion-associated and PKC-modulated changes in serine/threonine phosphorylation of p120-catenin. Biochemistry 42, 9195-204
**60** Oh M, et al. (2009) GSK-3 phosphorylates delta-catenin and negatively regulates its stability via ubiquitination/proteosome-mediated proteolysis. J Biol Chem 284, 28579-89
**61** Bilimoria PM, et al. (2010) A JIP3-regulated GSK3β/DCX signaling pathway restricts axon branching. J Neurosci 30, 16766-76
**62** Cernotta N, Clocchiatti A, Florean C, Brancolini C (2011) Ubiquitin-dependent degradation of HDAC4, a new regulator of random cell motility. Mol Biol Cell 22, 278-89
**63** Clayton EL, et al. (2010) Dynamin I phosphorylation by GSK3 controls activity-dependent bulk endocytosis of synaptic vesicles. Nat Neurosci 13, 845-51
**64** García-Alvarez G, et al. (2007) Glycogen synthase kinase-3beta binds to E2F1 and regulates its transcriptional activity. Biochim Biophys Acta 1773, 375-82
**65** Medunjanin S, et al. (2005) Glycogen synthase kinase-3 interacts with and phosphorylates estrogen receptor alpha and is involved in the regulation of receptor activity. J Biol Chem 280, 33006-14
**66** Maurer U, et al. (2006) Glycogen synthase kinase-3 regulates mitochondrial outer membrane permeabilization and apoptosis by destabilization of MCL-1. Mol Cell 21, 749-60
**67** Hagen T, et al. (2006) FRAT1, a substrate-specific regulator of glycogen synthase kinase-3 activity, is a cellular substrate of protein kinase A. J Biol Chem 281, 35021-9
**68** Humphrey RK, Yu SM, Flores LE, Jhala US (2010) Glucose regulates steady-state levels of PDX1 via the reciprocal actions of GSK3 and AKT kinases. J Biol Chem 285, 3406-16
**69** Tyagarajan SK, et al. (2011) Regulation of GABAergic synapse formation and plasticity by GSK3beta-dependent phosphorylation of gephyrin. Proc Natl Acad Sci U S A 108, 379-84
**70** Happel N, Stoldt S, Schmidt B, Doenecke D (2009) M phase-specific phosphorylation of histone H1.5 at threonine 10 by GSK-3. J Mol Biol 386, 339-50
**71** Vega RB, et al. (2002) Multiple domains of MCIP1 contribute to inhibition of calcineurin activity. J Biol Chem 277, 30401-7
**72** Zhao D, Zheng HQ, Zhou Z, Chen C (2010) The Fbw7 tumor suppressor targets KLF5 for ubiquitin-mediated degradation and suppresses breast cell proliferation. Cancer Res 70, 4728-38
**73** Flügel D, Görlach A, Michiels C, Kietzmann T. Glycogen synthase kinase 3 phosphorylates hypoxia-inducible factor 1alpha and mediates its destabilization in a VHL-independent manner. Mol Cell Biol. 2007 May;27(9):3253-65.
**74** Kang T, et al. (2008) GSK-3 beta targets Cdc25A for ubiquitin-mediated proteolysis, and GSK-3 beta inactivation correlates with Cdc25A overproduction in human cancers. Cancer Cell 13, 36-47
**75** Gauthier E, et al. (2005) Protein kinase A-dependent phosphorylation of Lutheran/basal cell adhesion molecule glycoprotein regulates cell adhesion to laminin alpha5. J Biol Chem 280, 30055-62
**76** Timm T, Balusamy K, Li X, Biernat J, Mandelkow E, Mandelkow EM. Glycogen synthase kinase (GSK) 3beta directly phosphorylates Serine 212 in the regulatory loop and inhibits microtubule affinity-regulating kinase (MARK) 2. J Biol Chem. 2008 Jul 4;283(27):18873-82.
**77** Barbash O, et al. (2008) Mutations in Fbx4 inhibit dimerization of the SCF(Fbx4) ligase and contribute to cyclin D1 overexpression in human cancer. Cancer Cell 14, 68-78
**78** Wang X, She H, Mao Z (2009) Phosphorylation of neuronal survival factor MEF2D by glycogen synthase kinase 3beta in neuronal apoptosis. J Biol Chem 284, 32619-26
**79** Badorff C, Seeger FH, Zeiher AM, Dimmeler S (2005) Glycogen synthase kinase 3beta inhibits myocardin-dependent transcription and hypertrophy induction through site-specific phosphorylation. Circ Res 97, 645-54
**80** Chen Y, et al. (2011) Dual Phosphorylation of Suppressor of Fused (Sufu) by PKA and GSK3{beta} Regulates Its Stability and Localization in the Primary Cilium. J Biol Chem 286, 13502-11
**81** Hergovich A, et al. (2006) Priming-dependent phosphorylation and regulation of the tumor suppressor pVHL by glycogen synthase kinase 3. Mol Cell Biol 26, 5784-96
**82** A.E Aplin, G.M Gibb, J.S Jacobsen, J.M Gallo and B.H Anderton, In vitro phosphorylation of the cytoplasmic domain of the amyloid precursor protein by glycogen synthase kinase-3β. J. Neurochem., 67 (1996), pp. 699-707
**83** Byeon IJ, et al. (2005) Sequential phosphorylation and multisite interactions characterize specific target recognition by the FHA domain of Ki67. Nat Struct Mol Biol 12, 987-93
**84** Yin L, Wang J, Klein PS, Lazar MA (2006) Nuclear receptor Rev-erbalpha is a critical lithium-sensitive component of the circadian clock. Science 311, 1002-5
**85** Dietz KN, et al. (2011) Identification of serines 201 and 209 as sites of Pax3 phosphorylation and the altered phosphorylation status of Pax3-FOXO1 during early myogenic differentiation. Int J Biochem Cell Biol 43, 936-45
**86** Chiang MH, et al. (2009) Mechanism of Hypoxia-induced GCM1 Degradation: IMPLICATIONS FOR THE PATHOGENESIS OF PREECLAMPSIA. J Biol Chem 284, 17411-9
**87** Saurin AT, et al. (2008) The regulated assembly of a PKCepsilon complex controls the completion of cytokinesis. Nat Cell Biol 10, 891-901
**88** Heyd F, Lynch KW (2010) Phosphorylation-dependent regulation of PSF by GSK3 controls CD45 alternative splicing. Mol Cell 40, 126-37
**89** Taniuchi M, et al. Phosphorylation of nerve growth factor receptor proteins in sympathetic neurons and PC12 cells. In vitro phosphorylation by the cAMP-independent protein kinase FA/GSK-3. J. Biol. Chem., 261 (1986), pp. 13342-13349.
**90** Watanabe T, Noritake J, Kakeno M, Matsui T, Harada T, Wang S, Itoh N, Sato K, Matsuzawa K, Iwamatsu A, Galjart N, Kaibuchi K. Phosphorylation of CLASP2 by GSK-3beta regulates its interaction with IQGAP1, EB1 and microtubules. J Cell Sci. 2009;122(Pt 16):2969-79.
**91** Ghazaleh HA, et al. (2010) 14-3-3 Mediated regulation of the tumor suppressor protein, RASSF1A. Apoptosis 15, 117-27
**92** Q.M Wang, I.K Park, C.J Fiol, P.J Roach and A.A DePaoli-Roach, Isoform differences in substrate recognition by glycogen synthase kinases 3α and 3β in the phosphorylation of phosphatase inhibitor 2. Biochemistry, 33 (1994), pp. 143-147
**93** Chen T, et al. (2009) Identification of Zinc-finger BED Domain-containing 3 (Zbed3) as a Novel Axin-interacting Protein That Activates Wnt/{beta}-Catenin Signaling. J Biol Chem 284, 6683-9
**94** Linding R, et al. (2007) Systematic discovery of in vivo phosphorylation networks. Cell 129, 1415-26
**95** Yook JI, et al. (2005) Wnt-dependent regulation of the E-cadherin repressor snail. J Biol Chem 280, 11740-8
**96** Avraham E, et al. (2005) Glycogen synthase kinase 3beta modulates synphilin-1 ubiquitylation and cellular inclusion formation by SIAH: implications for proteasomal function and Lewy body formation. J Biol Chem 280, 42877-86
**97** Cheng TS, et al. (2008) Glycogen Synthase Kinase 3 Interacts with and Phosphorylates the Spindle-associated Protein Astrin. J Biol Chem 283, 2454-2464
**98** Punga T, Bengoechea-Alonso MT, Ericsson J (2006) Phosphorylation and ubiquitination of the transcription factor sterol regulatory element-binding protein-1 in response to DNA binding. J Biol Chem 281, 25278-86
**99** Lucas F.R., et al. Inhibition of GSK-3beta leading to the loss of phosphorylated MAP-1 B is an early event in axonal remodeling induced by WNT-7a or lithium. J. Cell Sci., 111 (1998), pp. 1351-1361.
**100** Charvet C, et al. (2011) Phosphorylation of Tip60 by GSK-3 determines the induction of PUMA and apoptosis by p53. Mol Cell 42, 584-96
**101** Yeh TY, Sbodio JI, Chi NW (2006) Mitotic phosphorylation of tankyrase, a PARP that promotes spindle assembly, by GSK3. Biochem Biophys Res Commun 350, 574-9
**102** Morisco C, Seta K, Hardt SE, Lee Y, Vatner SF, Sadoshima J. Glycogen synthase kinase 3beta regulates GATA4 in cardiac myocytes. J Biol Chem. 2001; 276(30):28586-97.
**103** Danek El, et al. (2007) Glycogen synthase kinase-3 phosphorylates CdGAP at a consensus ERK 1 regulatory site. J Biol Chem 282, 3624-31
**104** Tolnay M, Juang YT, Tsokos GC (2002) Protein kinase A enhances, whereas glycogen synthase kinase-3 beta inhibits, the activity of the exon 2-encoded transactivator domain of heterogeneous nuclear ribonucleoprotein D in a hierarchical fashion. Biochem J 363, 127-36
**105** Hashimoto YK, Satoh T, Okamoto M, Takemori H (2008) Importance of autophosphorylation at Ser186 in the A-loop of salt inducible kinase 1 for its sustained kinase activity. J Cell Biochem 104, 1724-39
**106** Cai X, Li M, Vrana J, Schaller MD (2006) Glycogen synthase kinase 3- and extracellular signal-regulated kinase-dependent phosphorylation of paxillin regulates cytoskeletal rearrangement. Mol Cell Biol 26, 2857-68
**107** Jope RS, Johnson GV. The glamour and gloom of glycogen synthase kinase-3. Trends Biochem Sci. 2004 Feb;29(2):95-102.

## Claims

1. A method of detecting T-cell proliferation in a subject comprising the steps of;
a. obtaining a sample from said subject; and
b. measuring the level of expression, phosphorylation, activation and inactivation of total GSK-3β, p-GSK-3β Ser 9 phosphorylation site, PKC, P38 MAPK, Erk, PI-3K/Akt, in said sample;
c. comparing the levels to control levels determined from healthy subjects and measuring a significant deviation from said control levels;
wherein a decrease in T-cell proliferation is indicated when P38 MAPK, Erk and PI-3K/Akt, are inhibited and PKC is unaffected; or
wherein inhibition of T-cell proliferation is indicated when P38 MAPK, Erk and PI-3K/Akt, are unaffected and PKC inhibited, or
wherein upregulation of T-cell proliferation is indicated when P38 MAPK, Erk and PI-3K/Akt, are inhibited and PKC is activated or a decrease in the expression level of p-GSK-3β Ser 9 or total GSK-3β.

2. A diagnostic kit for the detection of pathological T-cell proliferation provided with means for measuring the level of expression, phosphorylation, activation and inactivation of GSK-3β, p-GSK-3β Ser 9 phosphorylation site, PKC, P38 MAPK, Erk, PI-3K/Akt, in combination, thereby providing a positive diagnosis of pathological T-cell proliferation.

3. A protein chip for the detection of pathological T-cell proliferation comprising an array of bound antibodies, biomarkers or antigens for determining the level of expression, phosphorylation, activation and inactivation of total GSK-3β, p-GSK-3β Ser 9 phosphorylation site, PKC, P38 MAPK, Erk, PI-3K/Akt.
